# EUROPEAN PATENT APPLICATION

(11) **EP 4 461 316 A1**
(43) Date of publication of application: **13.11.2024**
(21) Application number: 22922990.1
(22) Date of filing: 10.02.2022
(51) Int. Cl.: A61K 47/18, A61K 47/22, A61K 48/00, C07D 295/13, C07C 237/22, C07C 231/06

(54) **CATIONIC LIPID ANALOG, AND COMPOSITION AND USE THEREOF**

(30) Priority: 27.01.2022 CN 202210101998
(71) Applicant: Guangzhou Lide Biomedicine Technology Co., Ltd., Guangdong 510535 (CN)
(72) Inventor: LIU, Zhijia, Guangzhou, Guangdong 510000 (CN); CHEN, Yongming, Guangzhou, Guangdong 510000 (CN); HE, Zepeng, Guangzhou, Guangdong 510000 (CN); LE, Zhicheng, Guangzhou, Guangdong 510000 (CN)
(74) Representative: Zaboliene, Reda
(86) International application number: PCT/CN2022/075911
(87) International publication number: WO 2023/142167

(57) **Abstract**

A cationic lipid analogue, a composition and a use thereof. An aldehyde, an amine, a carboxylic acid and an isonitrile are adopted as raw materials to synthesize a cationic lipid analogue via the Ugi reaction. The synthesized cationic lipid analogue, together with a sterol, an auxiliary lipid and a polyethylene glycol lipid derivative, further forms a nanoparticle-like lipid composition, which can be used for drug delivery, including the delivery of small molecule drugs, nucleic acid drugs such as mRNA, DNA, siRNA, etc., protein/peptide drugs, and gene editing complexes such as mRNA/sgRNA, Cas9/sgRNA, etc. The nanoparticle-like lipid composition achieves mRNA expressions in different organs of animals through different administration methods, and thus can meet the application requirements of mRNA nucleic acid therapies, nucleic acid vaccines, gene editing, etc.

## Description

### TECHNICAL FIELD

The present disclosure relates to the technical field of biomedicine, and particularly to a cationic lipid analogue, a composition and a use thereof.

### BACKGROUND

Liposomes are fully enclosed multilayer vesicles formed by lipid bilayers such as phospholipid bilayers, and they can encapsulate both water-soluble and fat-soluble substances. The liposomes have characteristics of high targeting efficiency, controlled and sustained release, safety and non-toxicity. In recent years, by using liposomes and their analogues, the delivery technologies of molecular drugs are becoming more mature, and an increasing number of studies have shown the potential of various lipid nanoparticles in molecular drug therapies.

According to the charge properties, liposomes can be divided into cationic liposomes, neutral liposomes and anionic liposomes. Among them, the cationic liposomes have numerous advantages, such as natural degradation, non-toxicity, non-immunogenicity, repeatable transfection, etc. Cationic liposomes are usually used as gene carriers, which are complexed with DNA or RNA to protect the DNA or RNA from inactivation or degradation by nucleases and transfer the carried genes to specific sites. It is ensured that the transfection process is convenient, easy and reproducible. Through the liposome delivery system with high gene-delivery efficiency, specific genes can be properly expressed in specific organs in the body to achieve therapeutic applications. In prior art, developed gene therapy applications include nucleic acid therapies, nucleic acid vaccines, protein replacement therapies, gene editing, etc. In particular, with the rapid development of nucleic acid vaccines in recent years, messenger RNA vaccines have received more attention due to their simple preparation, high efficiency and stability.

At present, many researches on cationic lipid particles have been reported, and commercialized cationic lipid materials, such as cationic phospholipid DOTAP and ionizable cationic lipid DLin-MC3, are also available. However, problems such as low delivery efficiency *in vivo,* toxicity of cations to biological organisms, weak delivery targeting *in vivo,* etc. are still existed.

Given that a slight difference in the structures of cationic lipid analogues will lead to significant changes in their biological properties, it is crucial to develop a new cationic lipid analogue with high transfection efficiency *in vivo,* low toxicity and specific targeting.

### SUMMARY OF THE DISCLOSURE

An objective of the present disclosure is to overcome the deficiencies of the prior art and provide a cationic lipid analogue, a composition and a use thereof.

To achieve the above objective, the present disclosure provides the following technical solutions.

In a first aspect, the present disclosure relates to a cationic lipid analogue with a structure as shown in any one of formula (I) to formula (V): 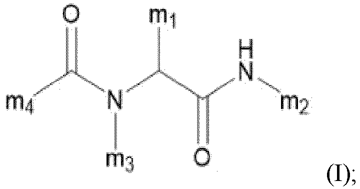 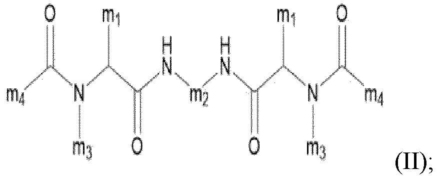 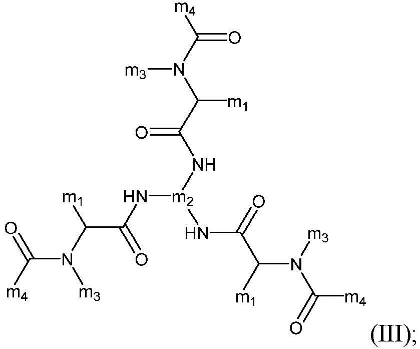 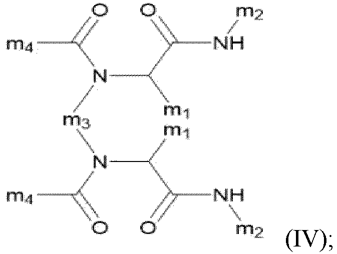 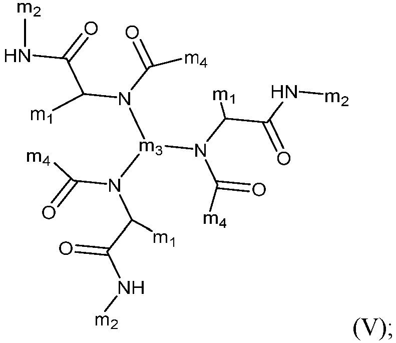
wherein each of m₁ is independently at least one selected from the group consisting of a hydrogen atom, a straight-chain alkyl, a branched-chain alkyl, a straight-chain alkenyl, a branched-chain alkenyl, a substituted alkynyl, a cycloalkyl, a phenyl, and a heteroatom-containing aromatic group;
each of m₂ is independently at least one selected from the group consisting of a straight-chain alkyl, a straight-chain alkenyl, a heteroatom-containing straight-chain alkyl, a heteroatom-containing straight-chain alkylene, a heteroatom-containing branched-chain alkyl, a heteroatom-containing branched-chain alkylene, a heterocycle-containing alkyl, a heterocycle-containing alkylene, an ester group-containing alkyl, and an ether group-containing alkyl;
each of m₃ is independently at least one selected from the group consisting of a straight-chain alkyl, a straight-chain alkenyl, a cycloalkyl, a heteroatom-containing straight-chain alkyl, a heteroatom-containing branched-chain alkyl, a heteroatom-containing branched-chain alkylene, a heterocycle-containing alkyl, a heteroatom-containing and aromatic ring-containing alkyl, a hydroxyl-containing alkyl, an ester alkyl, an ether alkyl, and a sulfonate substituted alkyl; and
each of m₄ is independently at least one selected from the group consisting of a straight-chain alkyl, a branched-chain alkyl, a straight-chain alkenyl, a branched-chain alkenyl, a straight-chain alkynyl, a heteroatom-containing straight-chain alkyl a heteroatom-containing branched-chain alkyl, and a heterocycle-containing alkyl.

The present disclosure adopts an aldehyde, an amine, a carboxylic acid, and an isonitrile as raw materials to synthesize a cationic lipid analogue via Ugi reaction. The cationic lipid analogue has high transfection efficiency *in vivo,* low toxicity, and specific targeting. And the cationic lipid analogue can achieve mRNA expression in different organs of animals through different administration methods, and thus can meet the application requirements of mRNA nucleic acid therapies, nucleic acid vaccines, gene editing, etc.

As a preferred embodiment of the cationic lipid analogue according to the present disclosure, in the formula, each of m₁ is independently selected from the group consisting of 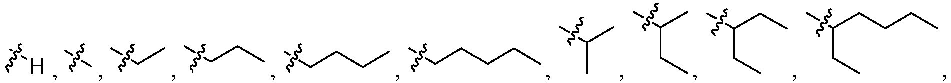 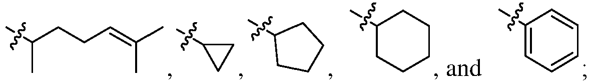 and/or
each of m₂ is independently selected from the group consisting of 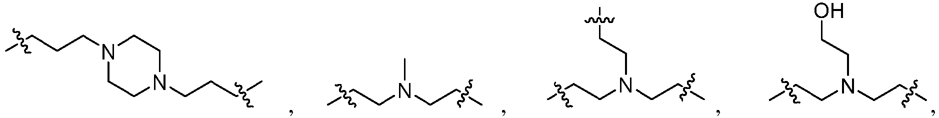 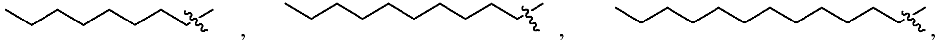 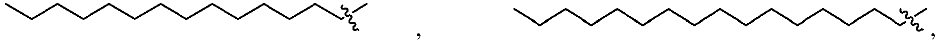 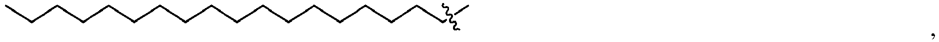 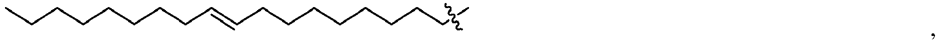 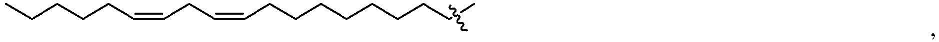 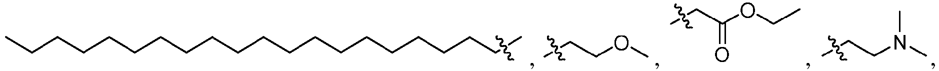 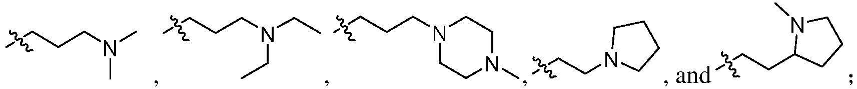 and/or
each of m₃ is independently selected from the group consisting of 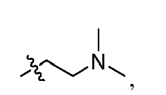 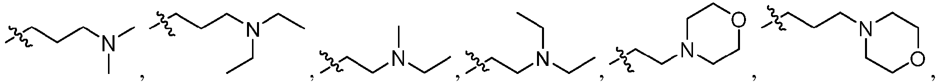 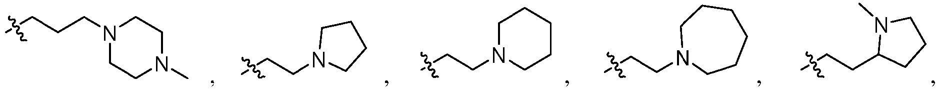 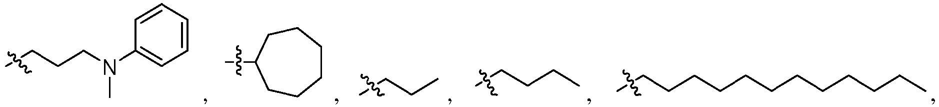 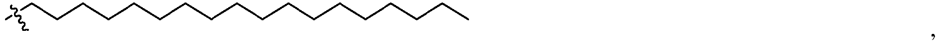 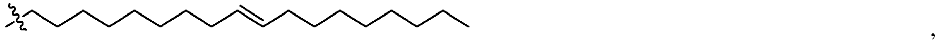 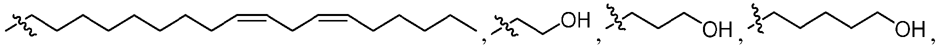 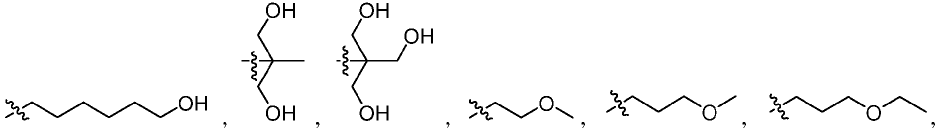 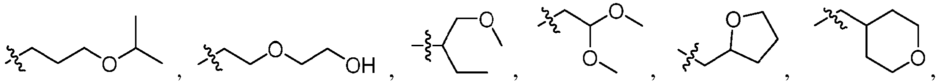 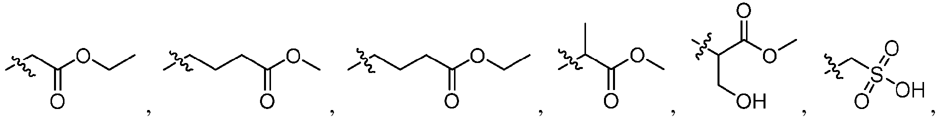 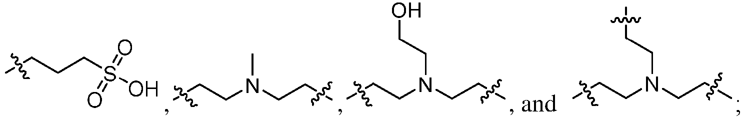 and/or
each of m₄ is independently selected from the group consisting of 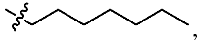 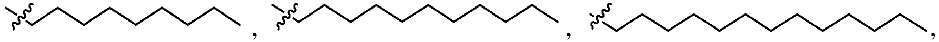 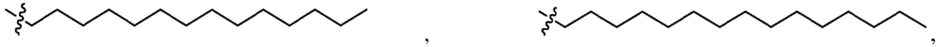 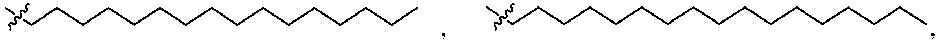 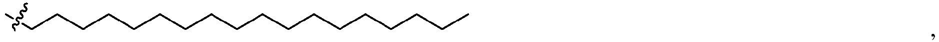 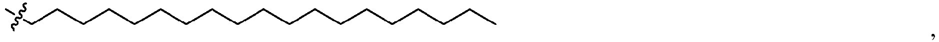 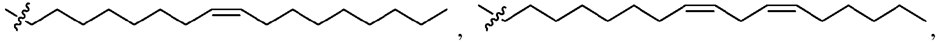 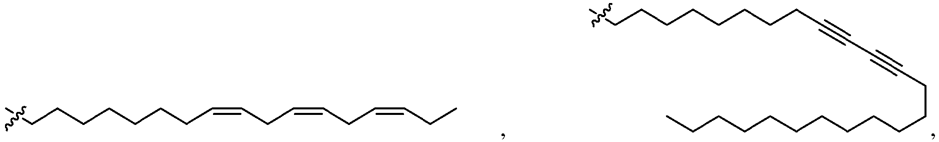 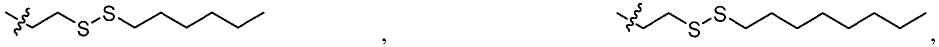 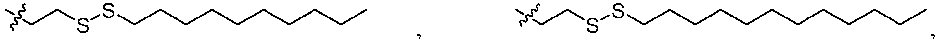 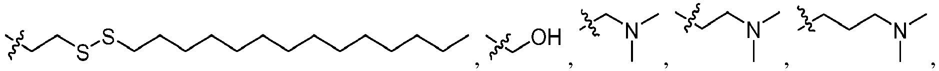 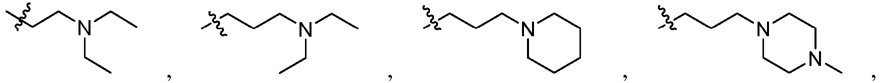 and

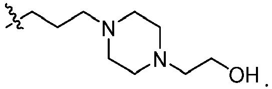

Preferably, each of m₁ is independently selected from the group consisting of 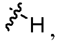 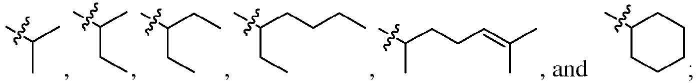 and/or
each of m₂ is independently selected from the group consisting of 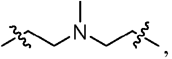 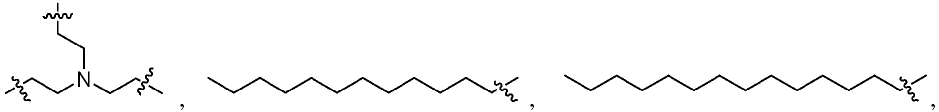 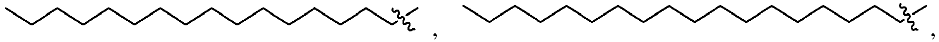 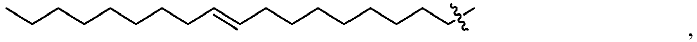 and 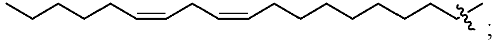 and/or
each of m₃ is independently selected from the group consisting of 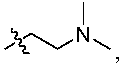 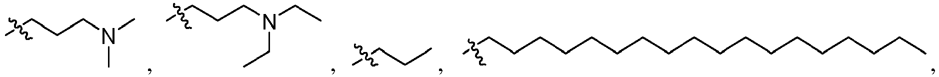 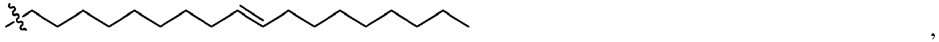 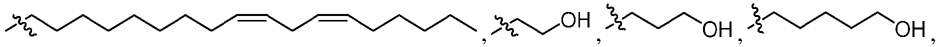 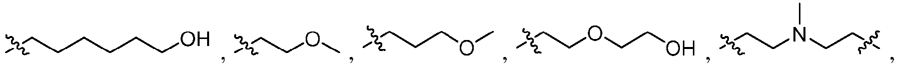 and 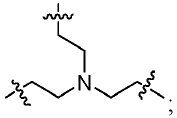 and/or
each of m₄ is independently selected from the group consisting of 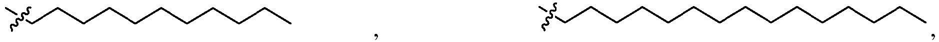 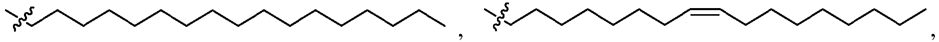 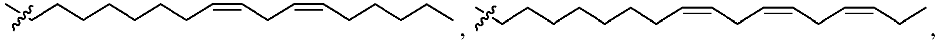 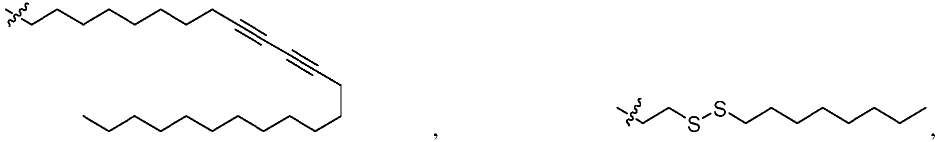 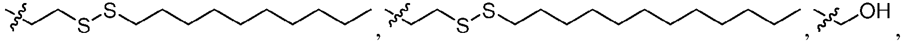 and

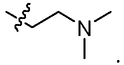

In a second aspect, the present disclosure relates to a method for preparing the cationic lipid analogue, which comprises steps of adding an aldehyde and an amine to an organic solution; allowing to react for 10-120 minutes before adding a carboxylic acid and an isonitrile in sequence; allowing to react at 4-60 °C for 6-72 hours; and performing a separation and a purification to obtain a product.

Preferably, a molar ratio of the aldehyde, the amine, the carboxylic acid and the isonitrile is (0.1-1.5): (0.1-1.5): (0.1-1.5): (0.1-1.5); more preferably, the molar ratio is 1: (0.33-1): 1: (0.33-1).

The cationic lipid analogue of the present disclosure is synthesized under a mild condition in a simple synthetic process, and has a good stability.

As a preferred embodiment of the method for preparing the cationic lipid analogue of the present disclosure, the isonitrile is one of a monofunctional isonitrile, a bifunctional isonitrile, or a trifunctional isonitrile.

Preferably, the isonitrile is any one of the following compounds, I3 to I38:

| | | | | | |
|---|---|---|---|---|---|
| I3 | 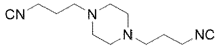 | I8 | 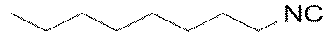 | I31 | 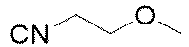 |
| I4 | 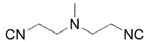 | I10 | 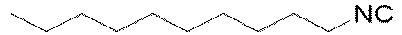 | I32 | 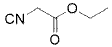 |
| | | I12 | 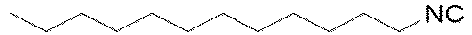 | | |
| I5 | 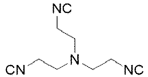 | I14 | 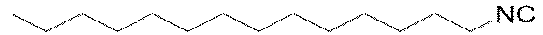 | I33 | 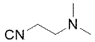 |
| | | I16 | 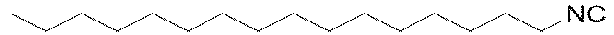 | | |
| I6 | 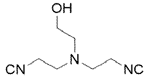 | I18 | 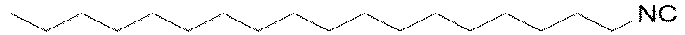 | I34 | 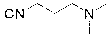 |
| | | I18-1 | 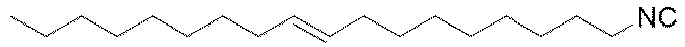 | I35 | 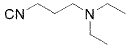 |
| | | I18-2 | 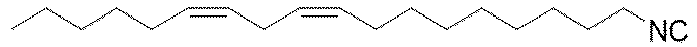 | | |
| | | I20 | 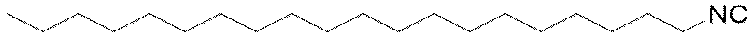 | I36 | 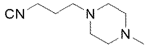 |
| | | | | I37 | 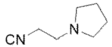 |
| | | | | I38 | 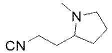 |

As a preferred embodiment of the method for preparing the cationic lipid analogue of the present disclosure, the aldehyde is a monofunctional aldehyde.

Preferably, the aldehyde is any one of the following compounds, A1 to A3 5:

| | | | | | |
|---|---|---|---|---|---|
| A1 | 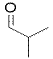 | A21 | 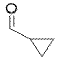 | A30 | 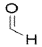 |
| A2 | 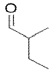 | A22 | 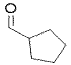 | A31 | 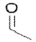 |
| A3 | 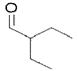 | A23 | 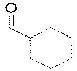 | A32 | 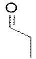 |
| A4 | 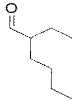 | A24 | 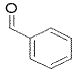 | A33 | 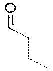 |
| A5 | 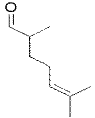 | | | A34 | 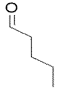 |
| | | | | A35 | 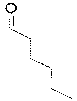 |

As a preferred embodiment of the method for preparing the cationic lipid analogue of the present disclosure, the amine is one of a monofunctional amine, a bifunctional amine, or a trifunctional amine.

As a preferred embodiment of the method for preparing the cationic lipid analogue of the present disclosure, the amine is any one of the following compounds, R1 to R53:

| | | | | | | | |
|---|---|---|---|---|---|---|---|
| R1 | 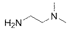 | R21 | 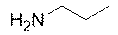 | R37 | 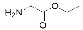 | R46 | 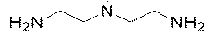 |
| R2 | 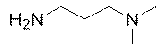 | R22 | 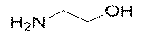 | R38 | 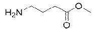 | | |
| R3 | 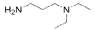 | R23 | 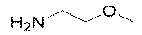 | R39 | 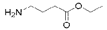 | R47 | 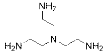 |
| R4 | 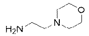 | R24 | 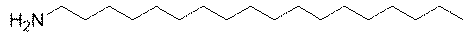 | | | | |
| | | R25 | 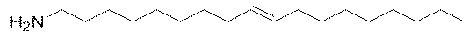 | R40 | 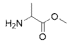 | R48 | 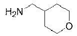 |
| R5 | 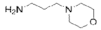 | R26 | 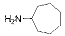 | | | | |
| R6 | 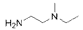 | R27 | 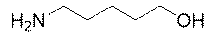 | R41 | 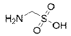 | R49 | 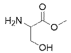 |
| R7 | 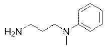 | R28 | 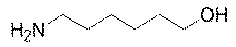 | R42 | 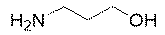 | R50 | 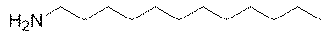 |
| R8 | 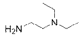 | R29 | 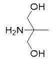 | R43 | 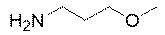 | R51 | 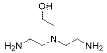 |
| R9 | 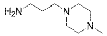 | R30 | 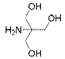 | R44 | 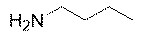 | | |
| R10 | 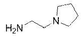 | | | R45 | 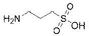 | R53 | 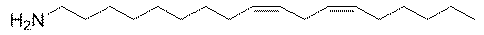 |
| R11 | 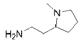 | R31 | 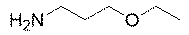 | | | | |
| | | R32 | 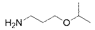 | | | | |
| R12 | 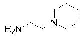 | | | | | | |
| | | R33 | 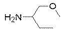 | | | | |
| R13 | 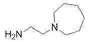 | R34 | 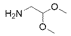 | | | | |
| | | R35 | 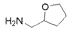 | | | | |
| | | R36 | 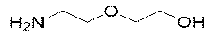 | | | | |

As a preferred embodiment of the method for preparing the cationic lipid analogue of the present disclosure, the carboxylic acid is any one of the following compounds, C8 to C30, or S 11 to S 19:

| | | | | | |
|---|---|---|---|---|---|
| C8 | 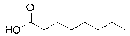 | C21 | 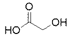 | S11 | 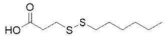 |
| C10 | 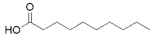 | | | S13 | 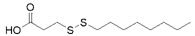 |
| C12 | 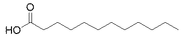 | C22 | 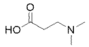 | | |
| C14 | 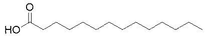 | | | S15 | 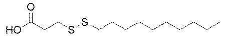 |
| C15 | 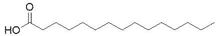 | C24 | 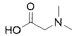 | | |
| C16 | 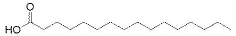 | C25 | 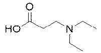 | S17 | 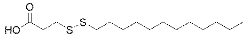 |
| C17 | 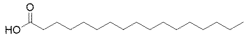 | | | S19 | 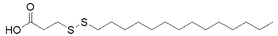 |
| C18 | 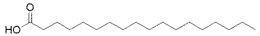 | C26 | 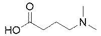 | | |
| C19 | 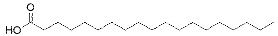 | C27 | 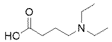 | | |
| C20 | 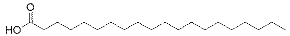 | | | | |
| C18-1 | 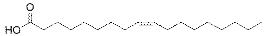 | C28 | 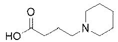 | | |
| C18-2 | 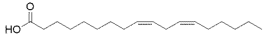 | C29 | 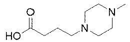 | | |
| C18-3 | 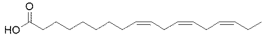 | | | | |
| C23 | 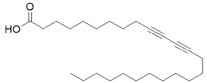 | C30 | 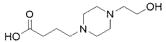 | | |

In a third aspect, the present disclosure relates to a composition comprising the cationic lipid analogue, and the composition comprises the cationic lipid analogue and at least one selected from the group consisting of a sterol, an auxiliary lipid, and a polyethylene glycol lipid derivative.

As a preferred embodiment of the composition comprising the cationic lipid analog of the present disclosure, the sterol comprises at least one selected from the group consisting of a cholesterol, a sitosterol (sitosterin), a stigmasterol (stigmasterin), and a cholesterol derivative; the auxiliary lipid is a non-cationic lipid, preferably, the auxiliary lipid is a neutral phospholipid, and more preferably, the auxiliary lipid is at least one selected from the group consisting of a phosphatidylcholine, a phosphatidylethanolamine, a sphingomyelin, and a ceramide.

As a preferred embodiment of the composition comprising the cationic lipid analogue of the present disclosure, a molar ratio of the cationic lipid analogue, the cholesterol, the auxiliary lipid, and the polyethylene glycol lipid derivative is (20-70): (20-50): (2-30): (0.1-20); preferably, the molar ratio is (40-60): (25-45): (5-15): (0.5-5).

In a fourth aspect, the present disclosure relates to a drug-loaded nanoparticle, which includes the composition comprising the cationic lipid analogue and a drug.

As a preferred embodiment of the drug-loaded nanoparticle of the present disclosure, the drug comprises at least one selected from the group consisting of a small molecule compound, a nucleic acid molecule, a protein or polypeptide molecule, and a gene editing complex.

Preferably, the nucleic acid molecule is at least one selected from the group consisting of messenger RNA (mRNA), transfer RNA (tRNA), dsRNA, shRNA, DNA, plasmid DNA, siRNA, antisense oligonucleotide, aiRNA, and miRNA; the gene editing complex is mRNA/sgRNA or Cas9/sgRNA.

Preferably, a mass ratio of the cationic lipid analogue to the nucleic acid molecule is (2-50): 1; preferably, the mass ratio of the cationic lipid analogue to nucleic acid molecule is (5-20): 1.

In a fifth aspect, the present disclosure relates to a method for preparing the drug-loaded nanoparticle, including the following steps:
(1) dissolving the composition comprising the cationic lipid analogue in an organic solution to obtain an organic phase;
(2) dissolving the nucleic acid molecule in a buffer solution to obtain an aqueous phase; a volume ratio of the aqueous phase to the organic phase is (2-5): 1;
(3) rapidly adding the aqueous phase to the organic phase (or rapidly adding the organic phase to the aqueous phase), mixing the aqueous phase and the organic phase evenly to obtain a mixture, and dialyzing the mixture to obtain the drug-loaded nanoparticle.

In a sixth aspect, the present disclosure relates to a use of the cationic lipid analogue, the composition comprising the cationic lipid analogue, and the drug-loaded nanoparticle in preparing, delivering or transporting a drug molecule or a nucleic acid vaccine.

Compared with the prior art, the present disclosure has the following beneficial effects:

The present disclosure adopts an aldehyde, an amine, a carboxylic acid and an isonitrile as raw materials to synthesize a cationic lipid analogue via the Ugi reaction. The cationic lipid analogue of the present disclosure is synthesized under a mild condition in a simple synthesis process, and has a good stability. The synthesized cationic lipid analogue, together with a sterol, an auxiliary lipid and a polyethylene glycol lipid derivative, further forms a nanoparticle-like lipid composition, which can be used for drug delivery, including the delivery of small molecule compounds, nucleic acid molecules such as mRNA, DNA, siRNA, etc., protein/peptide molecules, and gene editing complexes such as mRNA/sgRNA, Cas9/sgRNA, etc. The nanoparticle-like lipid composition achieves mRNA expressions in different organs of animals through different administration methods, and thus can meet the application requirements of mRNA nucleic acid therapies, nucleic acid vaccines, gene editing, etc.

### BRIEF DESCRIPTION OF THE DRAWINGS

Figure 1 is a proton nuclear magnetic resonance (¹H NMR) spectrum of A4I18R3C18-2 in Example 1.
Figure 2 is a mass spectrometry (MS) spectrum of A4I18R3C18-2 in Example 1.
Figure 3 is a ¹H NMR spectrum of A1I4R22C18-2 in Example 2.
Figure 4 is a MS spectrum of A1I4R22C18-2 in Example 2.
Figure 5 is a ¹H NMR spectrum of A 115R22S 15 in Example 3.
Figure 6 is a MS spectrum of A 115R22S 15 in Example 3.
Figure 7 is a ¹H NMR nuclear magnetic resonance spectrum of A1I32R46C18-2 in Example 4.
Figure 8 is a MS spectrum of A1I32R46C18-2 in Example 4.
Figure 9 is a ¹H NMR spectrum of A1I32R47C18-2 in Example 5.
Figure 10 is a MS spectrum of A1I32R47C18-2 in Example 5.
Figure 11 is a statistical graph of antibody titers after immunization with OVA-mRNA drug-loaded nanoparticles prepared with the compound of Example 1.
Figure 12 is a statistical graph of antibody titers after immunization with OVA-mRNA drug-loaded nanoparticles prepared with the compound of Example 2.

### DETAILED DESCRIPTION

In order to better illustrate the objective, the technical solutions and the advantages, the present disclosure will be further elaborated below in conjunction with specific embodiments. Those skilled in the art should understand that the specific embodiments described herein are only used for explaining the present disclosure and are not intended to limit the present disclosure.

Unless otherwise specified, all experimental methods used in the Examples are conventional methods. And all materials, reagents, etc. used can be obtained from commercial channels unless otherwise specified.

### Example 1: A cationic lipid analogue

A cationic lipid analogue was synthesized according to formula (I), and thus a product had a structural formula of

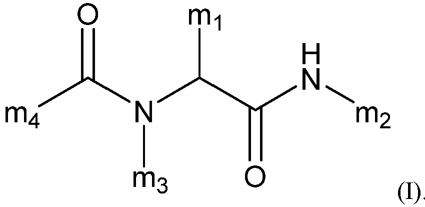

The specific preparation method was as follows:
1.0 mmol aldehyde and 1.0 mmol amine were added to 0.5 mL methanol solution at room temperature and reacted at room temperature for 60 minutes, then 1.0 mmol carboxylic acid was added to react at room temperature for 60 minutes, and then 1.0 mmol isonitrile was added to react at 40 °C for 24 hours. After the reaction was completed, a product was separated and purified by a chromatography column, where the mobile phase was a mixture of methanol and dichloromethane.

The cationic lipid analogue with a structure of formula (I) was synthesized according to the following synthetic route, 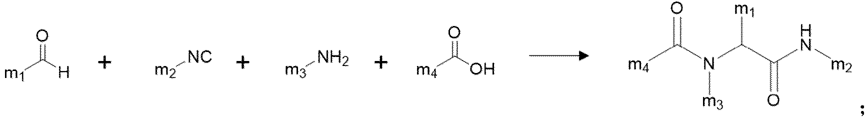 wherein the aldehyde was any one of A1, A4, or A23; the isonitrile was I18; the amine was any one of R1, R2, or R3; the carboxylic acid was any one of C18, C18-1, C18-2, C18-3, or C23.

There were 45 kinds of cationic lipid analogues synthesized by the above preferred compounds, and they were listed as follows:
A1I18R1C18, A1I18R1C18-1, A1I18R1C18-2, A1I18R1C18-3, A1I18R1C23, A1I18R2C18, A1I18R2C18-1, A1I18R2C18-2, A1I18R2C18-3, A1I18R2C23, A1I18R3C18, A1I18R3C18-1, A1I18R3C18-2, A1I18R3C18-3, A1I18R3C23, A4I18R1C18, A4I18R1C18-1, A4I18R1C18-2, A4I18R1C18-3, A4I18R1C23, A4I18R2C18, A4I18R2C18-1, A4I18R2C18-2, A4I18R2C18-3, A4I18R2C23, A4I18R3C18, A4I18R3C18-1, A4I18R3C18-2, A4I18R3C18-3, A4I18R3C23, A23I18R1C18, A23I18R1C18-1, A23I18R1C18-2, A23I18R1C18-3, A23I18R1C23, A23I18R2C18, A23I18R2C18-1, A23I18R2C18-2, A23I18R2C18-3, A23I18R2C23, A23I18R3C18, A23I18R3C18-1, A23I18R3C18-2, A23I18R3C18-3, and A23I18R3C23.

The aldehyde was any one of A1, A4, or A23; the isonitrile was I18; the amine was any one of R5, or R12; the carboxylic acid was any one of C18, or C18-2.

There were 12 kinds of cationic lipid analogues synthesized by the above preferred compounds, and they were listed as follows:
A1I18R5C18, A1I18R5C18-2, A1I18R12C18, A1I18R12C18-2, A4I18R5C18, A4I18R5C18-2, A4I18R12C18, A4I18R12C18-2, A23I18R5C18, A23I18R5C18-2, A23I18R12C18, and A23I18R12C18-2.

The aldehyde was A4 or A5; the isonitrile was I10, I14 or I18; the amine was R4, R6, R7, R8, R9, R10 or R11; and the carboxylic acid was C18 or C18-2.

There were 10 kinds of cationic lipid analogues synthesized by the above preferred compounds, and they were listed as follows:
A5I18R2C18-2, A4I10R2C18-2, A4I14R2C18-2, A4I18R4C18-2, A4I18R6C18-2, A4I18R7C18-2, A4I18R8C18-2, A4I18R9C18-2, A4I18R10C18-2, and A4I18R11C18-2.

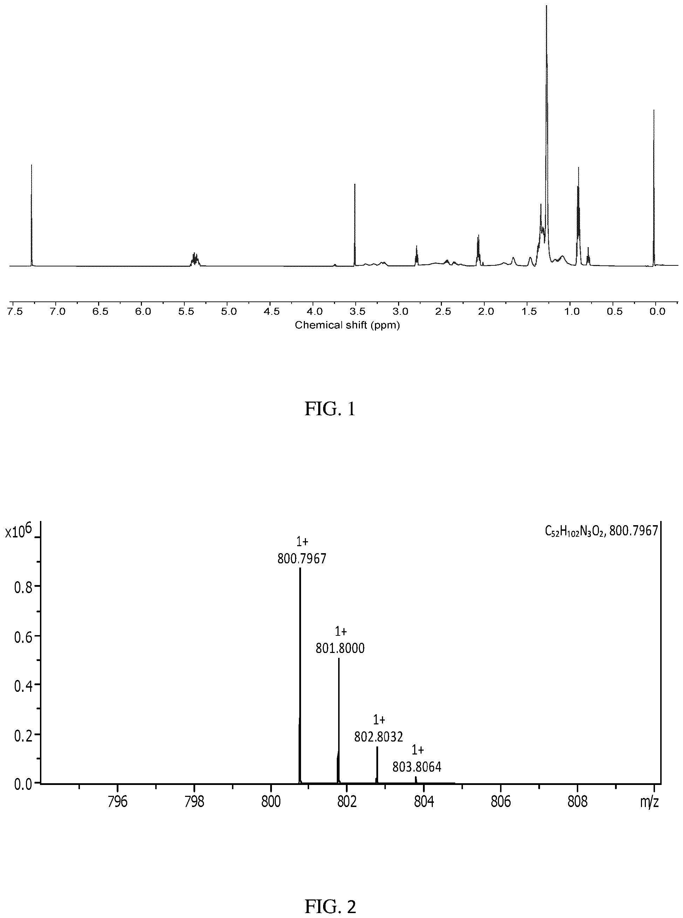

Among them, a ¹H NMR spectrum and a MS spectrum of A4I18R3C18-2 were shown in Figures 1 and 2, respectively.

### Example 2: A cationic lipid analogue

A cationic lipid analogue was synthesized according to formula (II), and thus a product had a structural formula of

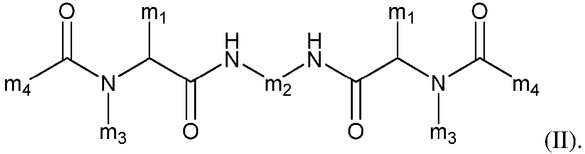

The specific preparation method was as follows:
1.0 mmol aldehyde and 1.0 mmol amine were added to 0.5 mL methanol solution at room temperature and reacted at room temperature for 60 minutes, then 1.0 mmol carboxylic acid was added to react at room temperature for 60 minutes, and then 0.5 mmol isonitrile was added to react at 40 °C for 24 hours. After the reaction was completed, a product was separated and purified by a chromatography column, where the mobile phase was a mixture of methanol and dichloromethane.

The cationic lipid analogue with a structure of formula (II) was synthesized according to the following synthetic route, 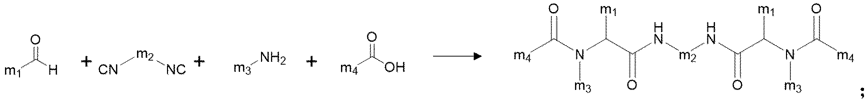 wherein the aldehyde was any one of A1, A4, A21, or A23; the isonitrile was I4; the amine was any one of R2, R13, R21, R22, or R23; and the carboxylic acid was any one of C18, C18-1, or C18-2.

There were 60 kinds of cationic lipid analogues synthesized by the above preferred compounds, and they were listed as follows:
A1I4R2C18, A1I4R2C18-1, A1I4R2C18-2, A1I4R13C18, A1I4R13C18-1, A1I4R13C18-2, A1I4R21C18, A1I4R21C18-1, A1I4R21C18-2, A1I4R22C18, A1I4R22C18-1, A1I4R22C18-2, A1I4R23C18, A1I4R23C18-1, A1I4R23C18-2, A4I4R2C18, A4I4R2C18-1, A4I4R2C18-2, A4I4R13C18, A4I4R13C18-1, A4I4R13C18-2, A4I4R21C18, A4I4R21C18-1, A4I4R21C18-2, A4I4R22C18, A4I4R22C18-1, A4I4R22C18-2, A4I4R23C18, A4I4R23C18-1, A4I4R23C18-2, A21I4R2C18, A21I4R2C18-1, A21I4R2C18-2, A21I4R13C18, A21I4R13C18-1, A21I4R13C18-2, A21I4R21C18, A21I4R21C18-1, A21I4R21C18-2, A21I4R22C18, A21I4R22C18-1, A21I4R22C18-2, A21I4R23C18, A21I4R23C18-1, A21I4R23C18-2, A23I4R2C18, A23I4R2C18-1, A23I4R2C18-2, A23I4R13C18, A23I4R13C18-1, A23I4R13C18-2, A23I4R21C18, A23I4R21C18-1, A23I4R21C18-2, A23I4R22C18, A23I4R22C18-1, A23I4R22C18-2, A23I4R23C18, A23I4R23C18-1, and A23I4R23C18-2.

The aldehyde was A1; the isonitrile was I4; the amine was any one of R27, R28, R29, R30, R31, R32, R33, R34, R35, R36, R42, R43, or R48; and the carboxylic acid was C18-2.

There were 13 kinds of cationic lipid analogues synthesized by the above preferred compounds, and they were listed as follows:
A1I4R27C18-2, A1I4R28C18-2, A1I4R29C18-2, A1I4R30C18-2, A1I4R31C18-2, A1I4R32C18-2, A1I4R33C18-2, A1I4R34C18-2, A1I4R35C18-2, A1I4R36C18-2, A1I4R42C18-2, A1I4R43C18-2, and A1I4R48C18-2.

The aldehyde was A1; the isonitrile was I4; the amine was R22; and the carboxylic acid was any one of C12, C14, C16, S11, S13, S15, S17, or S19.

There were 8 kinds of cationic lipid analogues synthesized by the above preferred compounds, and they were listed as follows:
A1I4R22C12, A1I4R22C14, A1I4R22C16, A1I4R22S11, A1I4R22S13, A1I4R22S15, A1I4R22S17, and A1I4R22S19.

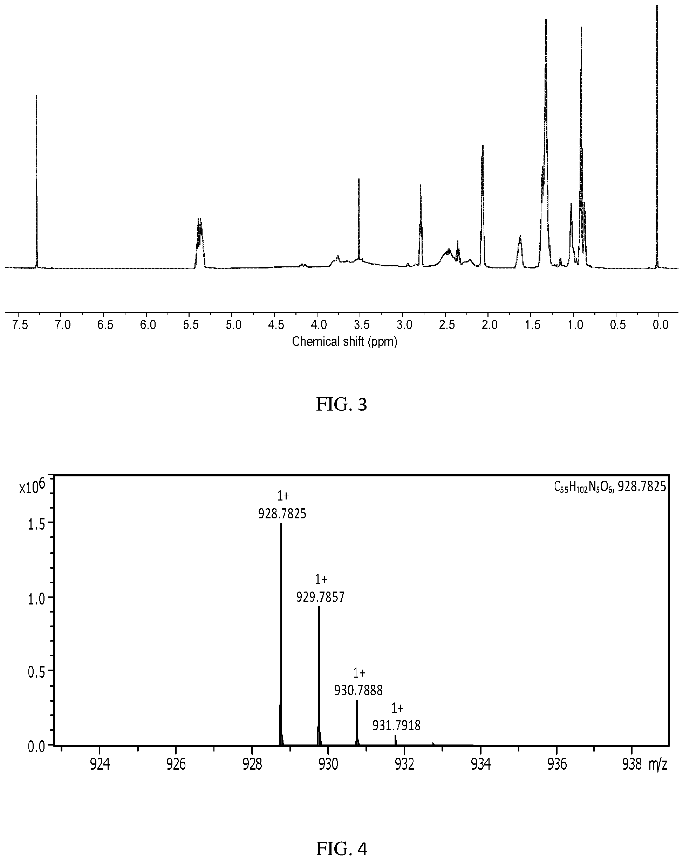

Among them, a ¹H NMR spectrum and a MS spectrum of A1I4R22C18-2 were shown in Figures 3 and 4, respectively.

### Example 3: A cationic lipid analogue

A cationic lipid analogue was synthesized according to formula (III), and thus a product had a structural formula of

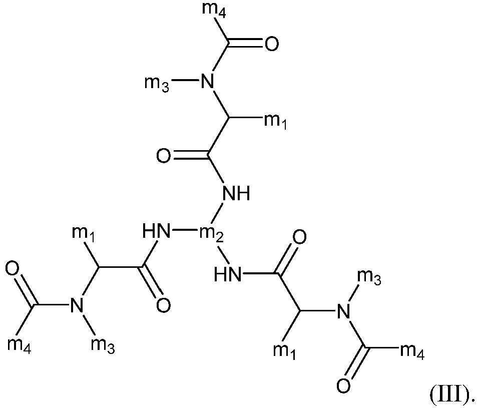

The specific preparation method was as follows:
1.0 mmol aldehyde and 1.0 mmol amine were added to 0.5 mL methanol solution at room temperature and reacted at room temperature for 60 minutes, then 1.0 mmol carboxylic acid was added to react at room temperature for 60 minutes, and then 0.33 mmol isonitrile was added to react at 40 °C for 24 hours. After the reaction was completed, a product was separated and purified by a chromatography column, where the mobile phase was a mixture of methanol and dichloromethane.

The cationic lipid analogue with a structure of formula (III) was synthesized according to the following synthetic route, 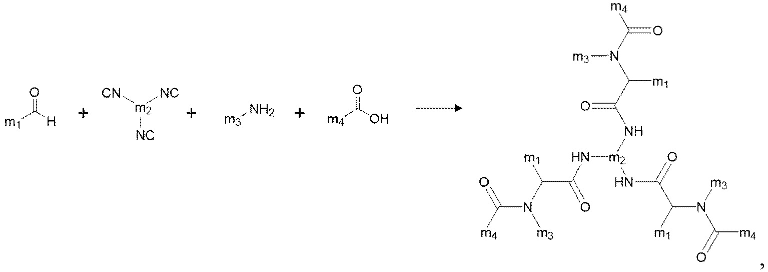 wherein the aldehyde was A1; the isonitrile is I5; the amine was R2, R13, R21, R22, R23, R26, R27, R28, R29, R30, R31, R32, R33, R34, R35, R36, R37, R38, R39, R40, R41 or R50; and the carboxylic acid was C8, C10, C12, C14, C16, C18, C18-2, C21, S11, S13, S15, S17 or S19.

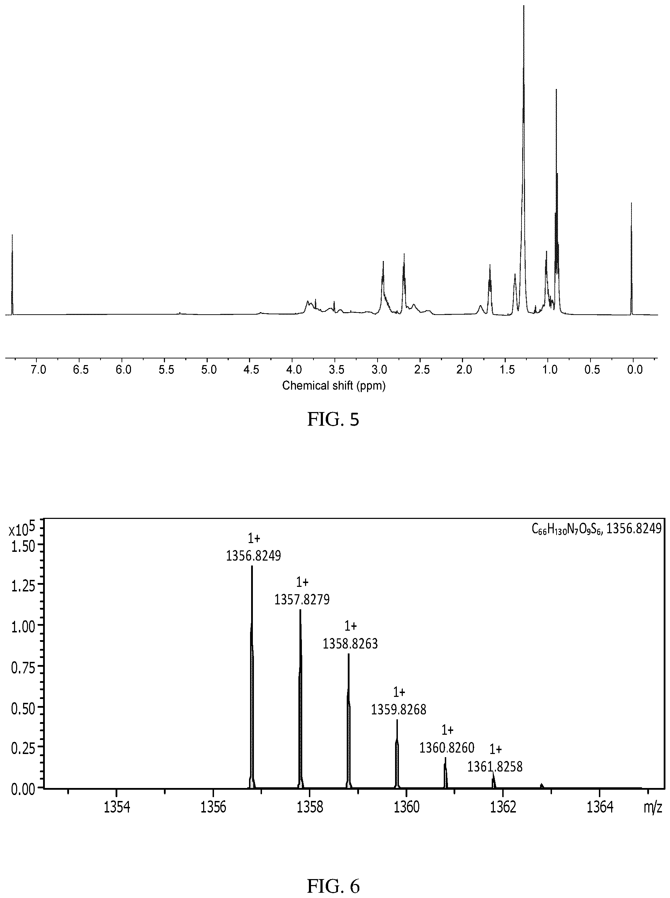

There were 29 kinds of cationic lipid analogues synthesized by the above preferred compounds, and they were listed as follows:
A1I5R22C8, A1I5R22C10, A1I5R22C12, A1I5R22C14, A1I5R22C16, A1I5R22C18, A1I5R22C18-2, A1I5R22S11, A1I5R22S13, A1I5R22S15, A1I5R22S17, A1I5R22S19, A1I5R26S15, A1I5R27S15, A1I5R28S15, A1I5R29S15, A1I5R30S15, A1I5R31S15, A1I5R32S15, A1I5R33S15, A1I5R34S15, A1I5R35S15, A1I5R36S15, A1I5R37S15, A1I5R38S15, A1I5R39S15, A1I5R40S15, A1I5R41S15, and A1I5R50C21.
Among them, a ¹H NMR spectrum and a MS spectrum of A1I5R22S15 were shown in Figures 5 and 6, respectively.

### Example 4: A cationic lipid analogue

A cationic lipid analogue was synthesized according to formula (IV), and thus a product had a structural formula of

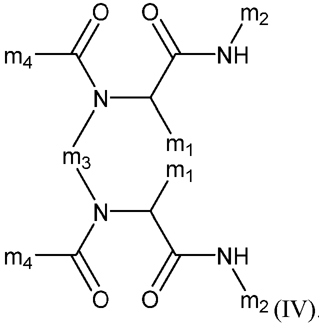

The specific preparation method was as follows:
1.0 mmol aldehyde and 0.5 mmol amine were added to 0.5 mL methanol solution at room temperature and reacted at room temperature for 60 minutes, then 1.0 mmol carboxylic acid was added to react at room temperature for 60 minutes, and then 1.0 mmol isonitrile was added to react at 40 °C for 24 hours. After the reaction was completed, a product was separated and purified by a chromatography column, where the mobile phase was a mixture of methanol and dichloromethane.

The cationic lipid analogue with a structure of formula (IV) was synthesized according to the following synthetic route, 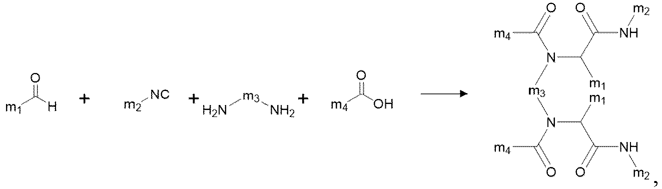 wherein the aldehyde was A1; the isonitrile was I10, 112,131 or I32; the amine was R46 or R51; and the carboxylic acid was C8 or C18-2.

There were 4 kinds of cationic lipid analogues synthesized by the above preferred compounds, and they were listed as follows:
A1I12R51C8, A1I10R51C18-2, A1I31R46C18-2, and A1I32R46C18-2.

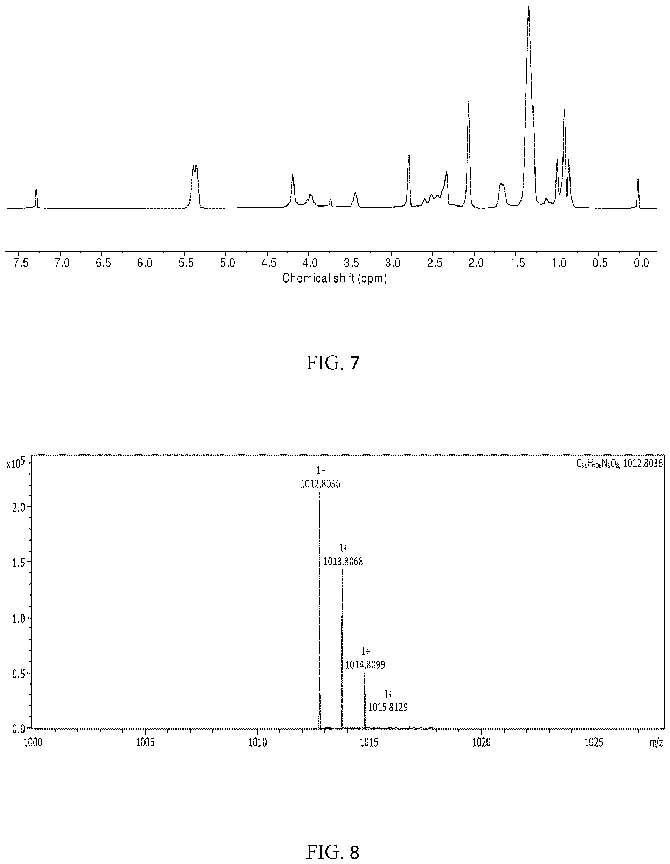

Among them, a ¹H NMR spectrum and a MS spectrum of A1I32R46C18-2 were shown in Figures 7 and 8, respectively.

### Example 5: A cationic lipid analogue

A cationic lipid analogue was synthesized according to formula (V), and thus a product had a structural formula of

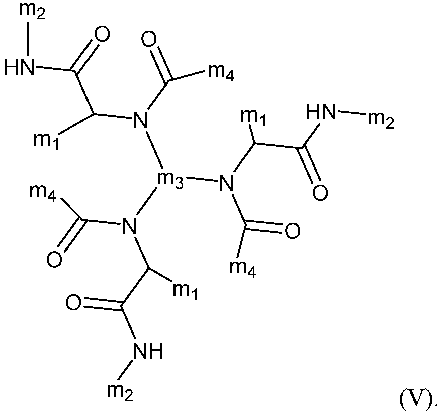

The specific preparation method was as follows:
1.0 mmol aldehyde and 0.33 mmol amine were added to 0.5 mL methanol solution at room temperature and reacted at room temperature for 60 minutes, then 1.0 mmol carboxylic acid was added to react at room temperature for 60 minutes, and then 1.0 mmol isonitrile was added to react at 40 °C for 24 hours. After the reaction was completed, a product was separated and purified by a chromatography column, where the mobile phase was a mixture of methanol and dichloromethane.

The cationic lipid analogue with a structure of formula (V) was synthesized according to the following synthetic route, 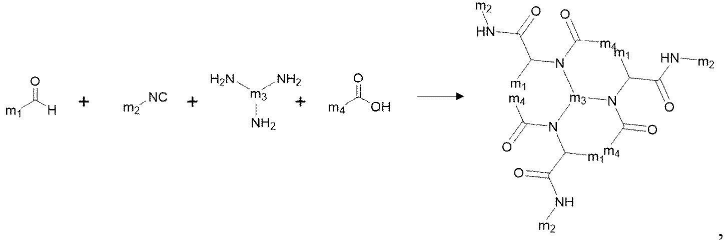 wherein the aldehyde was A1; the isonitrile is I12, I31 or I32; the amine was R47; and the carboxylic acid was C21 or C18-2.

There were 3 kinds of cationic lipid analogues synthesized by the above preferred compounds, and they were listed as follows:
A1I12R47C21, A1I31R47C18-2, and A1I32R47C18-2.

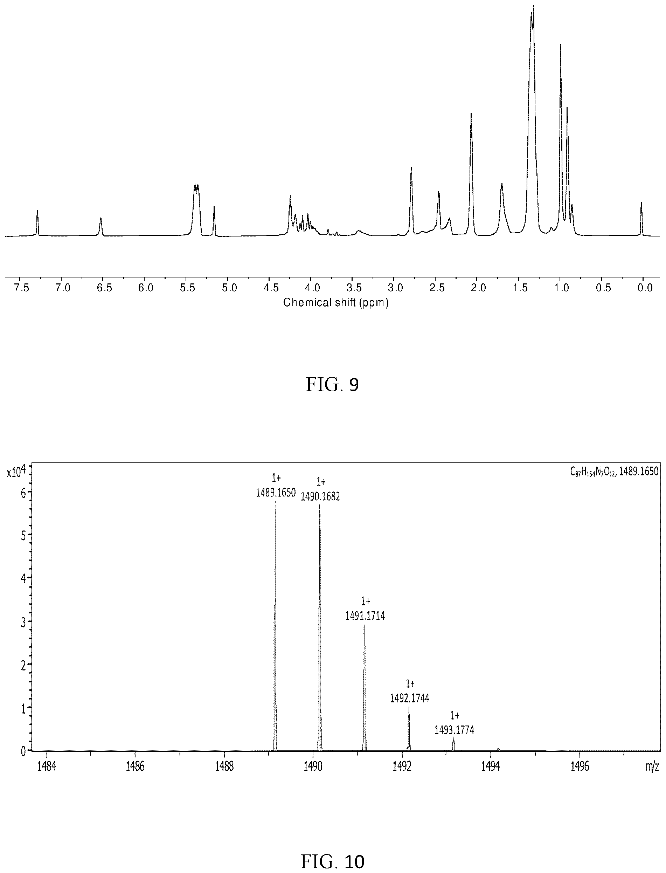

Among them, a ¹H NMR spectrum and a MS spectrum of A1I32R47C18-2 were shown in Figures 9 and 10, respectively.

### Example 6: A drug-loaded nanoparticle

The drug-loaded nanoparticle included a cationic lipid analogue, cholesterol, distearoylphosphatidylcholine (DSPC), and 1,2-dimyristoyl-rac-glycerol-3-methoxypolyethylene glycol (DMG-PEG),
wherein a molar ratio of the cationic lipid analogue: cholesterol: DSPC: DMG-PEG was 50:38.5:10:1.5.

In the drug-loaded nanoparticle, a mass ratio of the cationic lipid analogue to the nucleic acid drug, such as mRNA or siRNA, etc., was 11:1.

A preparation method of the mRNA-loaded nanoparticle was taken as an example.

A cationic lipid analogue, cholesterol, DSPC, and DMG-PEG were dissolved in an ethanol solution, and mixed in the above-mentioned molar ratio to obtain an organic phase. Then, an mRNA nucleic acid drug at a corresponding mass ratio was dissolved in a sodium acetate buffer solution (25 mM, pH = 3.0) to obtain an aqueous phase, so that a volume ratio of the aqueous phase to the organic phase was 3:1. Then, the aqueous phase was quickly added to the organic phase and mixed evenly, and the mixture was allowed to stand for 10 minutes. After that, the mixture was put into a 1000 Da dialysis bag, and dialyzed in a 1 × PBS solution at 4°C for 2 hours to obtain the nanoparticle that can be directly used for injection.

**Table 1 Characterization Data of the Lipid Nanoparticles Prepared in Example 6**

| Example | Name of the cationic lipid analogue | Diameter of the nanoparticles (nm) | PDI | Zeta potential (mV) | encapsulation efficiency |
|---|---|---|---|---|---|
| 1 | A4I18R3C18-2 | 83±9 | 0.12 | -3.2 | 75.3% |
| 2 | A1I4R22C18-2 | 133±6 | 0.17 | -5.5 | 62.1% |
| 3 | A1I5R22S15 | 144±7 | 0.22 | -7.2 | 65.4% |
| 4 | A1I32R46C18-2 | 110±11 | 0.20 | -2.5 | 67.2% |
| 5 | A1I32R47C18-2 | 150±9 | 0.21 | -3.1 | 60.5% |

### Example 7: A drug-loaded nanoparticle

The drug-loaded nanoparticle included a cationic lipid analogue, β-sitosterol, dioleoyl phosphatidylethanolamine (DOPE), and 1,2-dimyristoyl-rac-glycerol-3-methoxypolyethylene glycol (DMG-PEG),
wherein a molar ratio of the cationic lipid analogue: β-sitosterol: DOPE: DMG-PEG was 35:25:5:2. In the drug-loaded nanoparticle, a mass ratio of the cationic lipid analogue to the nucleic acid drug, such as mRNA or siRNA, was 5:1.

### Example 8: A drug-loaded nanoparticle

The drug-loaded nanoparticle included a cationic lipid analogue, stigmasterol, ceramide phosphorylcholine, and 1,2-dimyristoyl-rac-glycerol-3-methoxypolyethylene glycol (DMG-PEG),
wherein a molar ratio of the cationic lipid analogue: stigmasterol: ceramide phosphorylcholine: DMG-PEG was 55:40:12:2.5.

In the drug-loaded nanoparticle, a mass ratio of the cationic lipid analogue to the nucleic acid drug, such as mRNA or siRNA, was 15:1.

### Example 9: A drug-loaded nanoparticle

The drug-loaded nanoparticle included a cationic lipid analogue, cholesteryl hemisuccinate (cholesterol derivative), sphingomyelin, 1,2-dimyristoyl-rac-glycerol-3-methoxypolyethylene glycol (DMG-PEG),
wherein a molar ratio of the cationic lipid analogue: cholesteryl hemisuccinate: sphingomyelin: DMG-PEG was 60:45:15:3.5.

In the drug-loaded nanoparticle, a mass ratio of the cationic lipid analogue to the nucleic acid drug, such as mRNA or siRNA, was 18:1.

### Example 10: An animal experiment for detecting the in vivo expression level of the mRNA delivered by intravenous injection

Luciferase-mRNA was used as an mRNA model; and the cationic lipid analogues of Examples 1-5 were used to prepare drug-loaded nanoparticles according to the method of Example 6.

Detailed operating processes were as follows:
200 µL of nanoparticles containing 5 µg Luciferase-mRNA or the drug-loaded nanoparticles prepared above were injected into tail veins of 6-8 week-old C57BL/6 mice, and 200 µL of 1× PBS solution containing 3 mg D-Luciferin potassium salt was intraperitoneally injected into the mice 6 hours later. After waiting for 10 minutes, an *in vivo* expression level of the mRNA was evaluated by bioluminescence imaging.

The *in vivo* expression levels of the drug-loaded nanoparticles were shown in Tables 2 to 6.

**Table 2 In vivo expression levels of mRNA-loaded nanoparticles prepared by the compounds of Example 1 after intravenous injection**

| Name of the cationic lipid analogue | Fluorescence intensity of liver | Structural formula |
|---|---|---|
| A1I18R5C18 | 1.16E+03 | 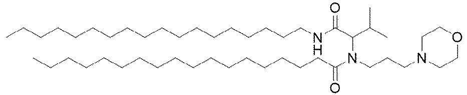 |
| A1I18R5C18-2 | 3.77E+03 | 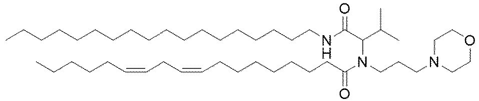 |
| A1I18R12C18 | 9.34E+02 | 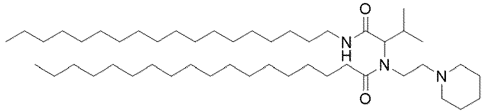 |
| A1I18R12C18-2 | 1.41E+03 | 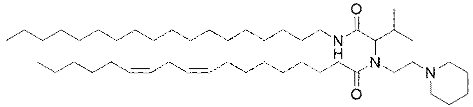 |
| A4I18R5C18 | 2.68E+03 | 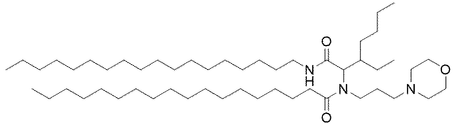 |
| A4I18R5C18-2 | 8.34E+03 | 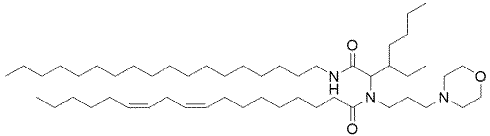 |
| A4I18R12C18 | 2.31E+03 | 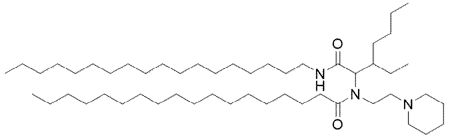 |
| A4I18R12C18-2 | 9.22E+03 | 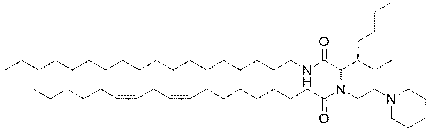 |
| A23I18R5C18 | 1.85E+03 | 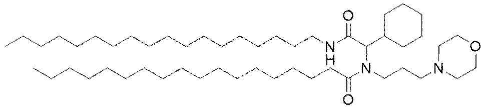 |
| A23I18R5C18-2 | 2.68E+03 | 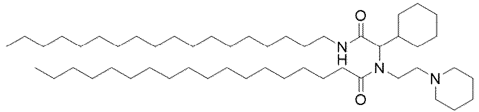 |
| A23I18R12C18 | 1.07E+03 | 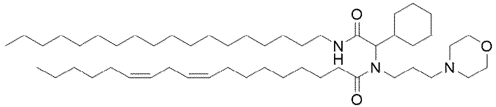 |
| A23I18R12C18-2 | 6.30E+03 | 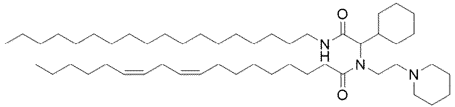 |
| A5I18R2C18-2 | 8.72E+05 | 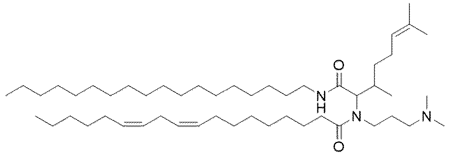 |
| A4I10R2C18-2 | 1.15E+06 | 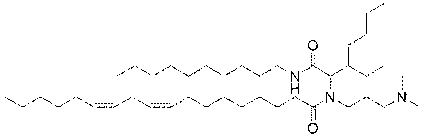 |
| A4I14R2C18-2 | 9.02E+05 | 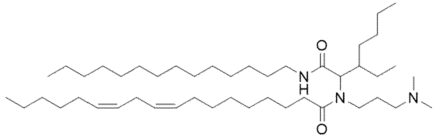 |
| A4I18R4C18-2 | 1.25E+04 | 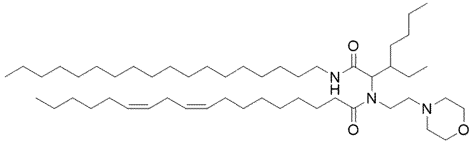 |
| A4I18R6C18-2 | 3.64E+04 | 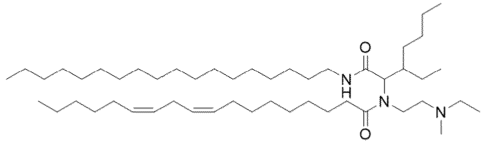 |
| A4I18R7C18-2 | 5.22E+03 | 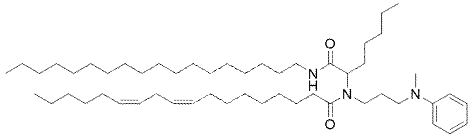 |
| A4I18R8C18-2 | 2.48E+04 | 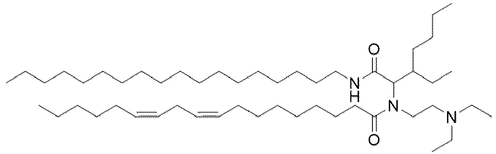 |
| A4I18R9C18-2 | 2.55E+03 | 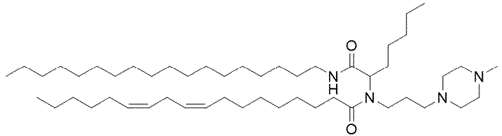 |
| A4I18R10C18-2 | 7.89E+05 | 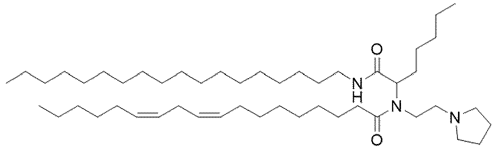 |
| A4I18R11C18-2 | 1.02E+06 | 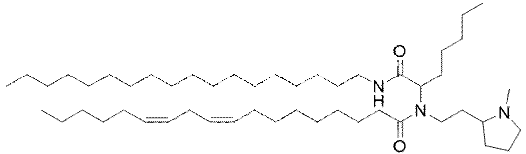 |
| A1I18R1C18 | 6.37E+03 | 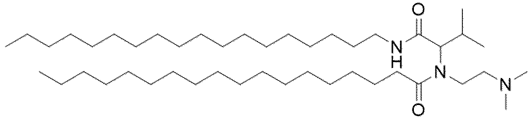 |
| A1I18R1C18-1 | 5.08E+05 | 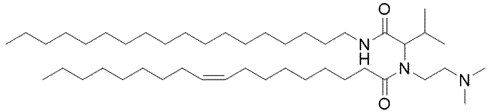 |
| A1I18R1C18-2 | 2.27E+05 | 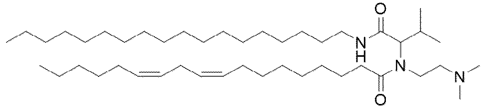 |
| A1I18R1C18-3 | 1.88E+06 | 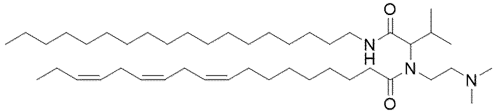 |
| A1I18R1C23 | 9.51E+05 | 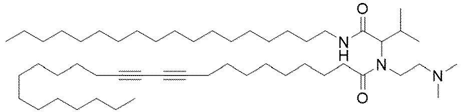 |
| A1I18R2C18 | 2.26E+04 | 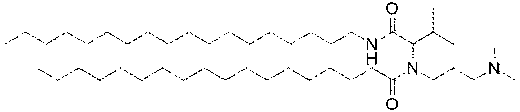 |
| A1I18R2C18-1 | 2.63E+04 | 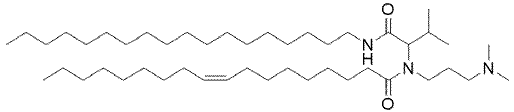 |
| A1I18R2C18-2 | 5.42E+04 | 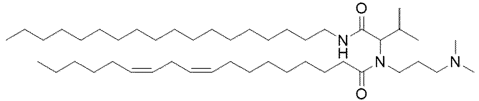 |
| A1I18R2C18-3 | 6.77E+04 | 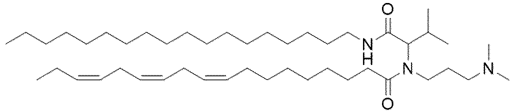 |
| A1I18R2C23 | 2.70E+05 | 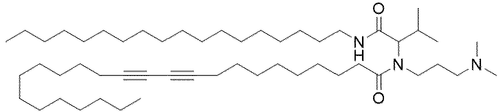 |
| A1I18R3C18 | 3.91E+03 | 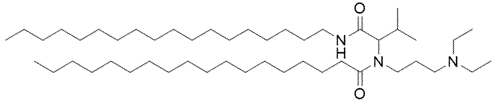 |
| A1I18R3C18-1 | 9.29E+03 | 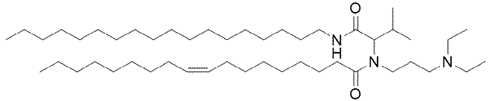 |
| A1I18R3C18-2 | 1.48E+05 | 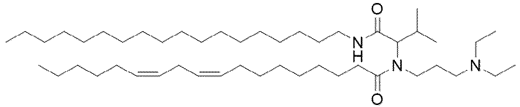 |
| A1I18R3C18-3 | 2.22E+04 | 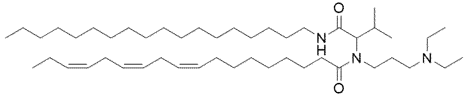 |
| A1I18R3C23 | 7.67E+04 | 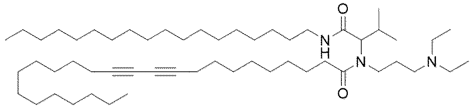 |
| A4I18R1C18 | 3.65E+05 | 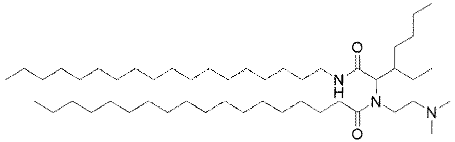 |
| A4I18R1C18-1 | 1.39E+07 | 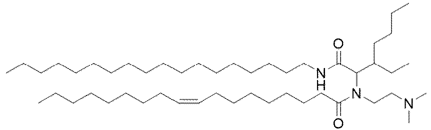 |
| A4I18R1C18-2 | 9.67E+06 | 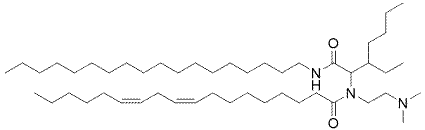 |
| A4I18R1C18-3 | 3.88E+06 | 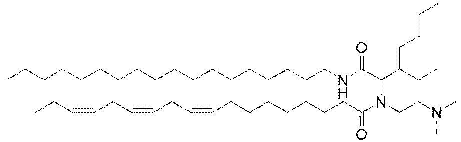 |
| A4I18R1C23 | 1.78E+06 | 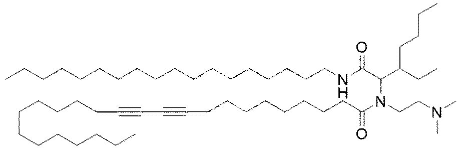 |
| A4I18R2C18 | 5.05E+05 | 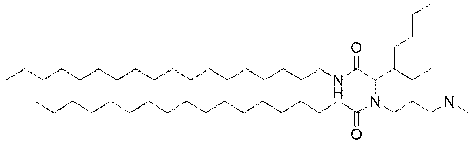 |
| A4I18R2C18-1 | 1.33E+07 | 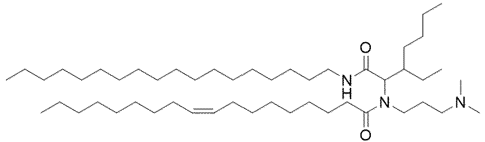 |
| A4I18R2C18-2 | 2.06E+07 | 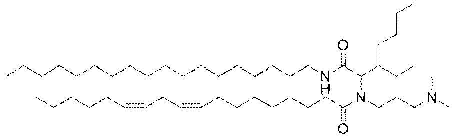 |
| A4I18R2C18-3 | 1.36E+07 | 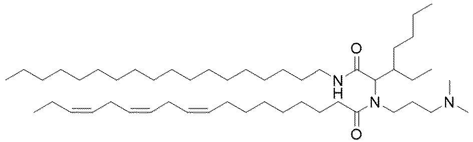 |
| A4I18R2C23 | 1.40E+07 | 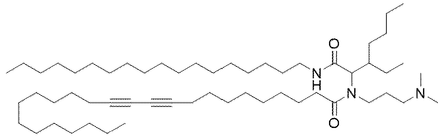 |
| A4I18R3C18 | 1.69E+06 | 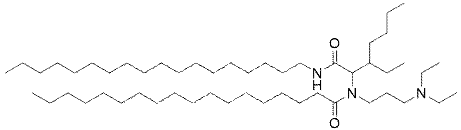 |
| A4I18R3C18-1 | 1.39E+07 | 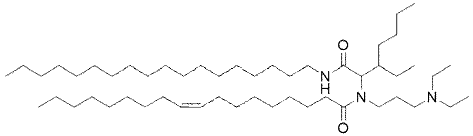 |
| A4I18R3C18-2 | 2.29E+07 | 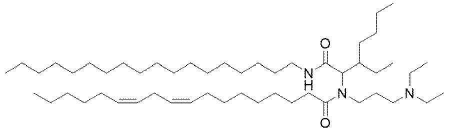 |
| A4I18R3C18-3 | 1.32E+07 | 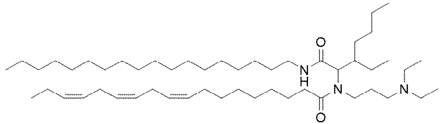 |
| A4I18R3C23 | 7.81E+06 | 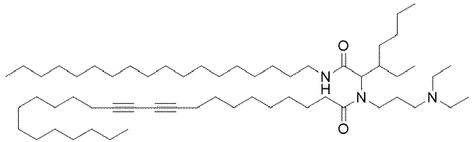 |
| A23I18R1C18 | 9.09E+04 | 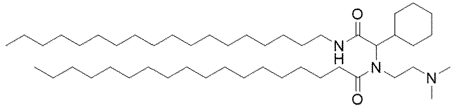 |
| A23I18R1C18-1 | 5.74E+06 | 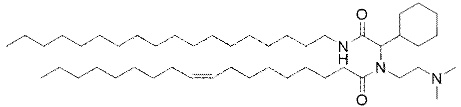 |
| A23I18R1C18-2 | 8.25E+06 | 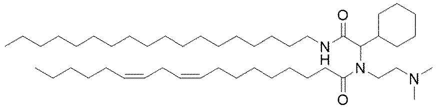 |
| A23I18R1C18-3 | 4.14E+06 | 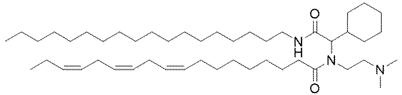 |
| A23I18R1C23 | 6.11E+06 | 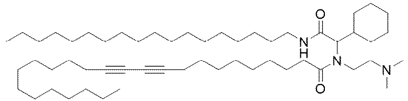 |
| A23I18R2C18 | 3.87E+05 | 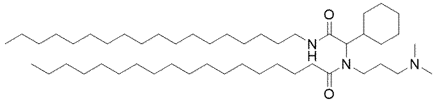 |
| A23I18R2C18-1 | 1.29E+06 | 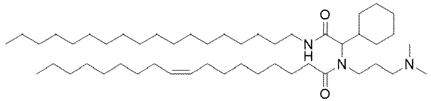 |
| A23I18R2C18-2 | 1.52E+06 | 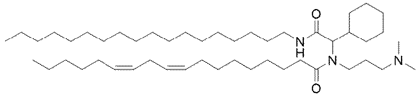 |
| A23I18R2C18-3 | 9.49E+05 | 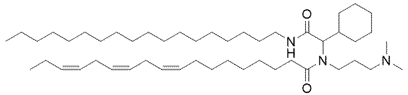 |
| A23I18R2C23 | 2.37E+06 | 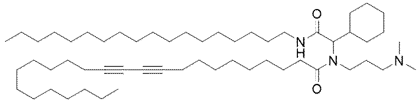 |
| A23I18R3C18 | 2.14E+03 | 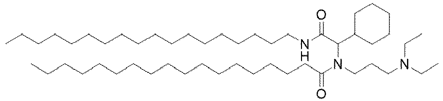 |
| A23I18R3C18-1 | 1.26E+06 | 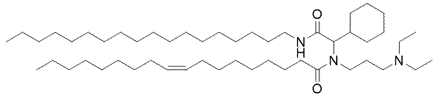 |
| A23I18R3C18-2 | 4.86E+06 | 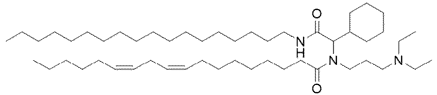 |
| A23I18R3C18-3 | 1.13E+06 | 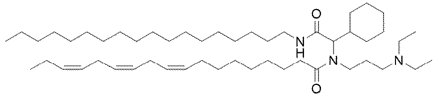 |
| A23I18R3C23 | 7.89E+05 | 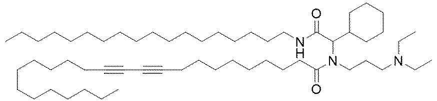 |

**Table 3 In vivo expression levels of mRNA-loaded nanoparticles prepared by the compounds of Example 2 after intravenous injection**

| Name of the cationic lipid analogues | Fluorescence intensity of spleen | Structural formula |
|---|---|---|
| A1I4R2C18 | 1.79E+04 | 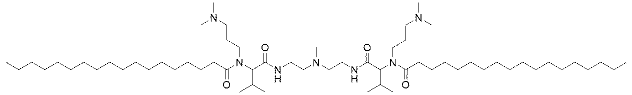 |
| A1I4R2C18-1 | 8.80E+02 | 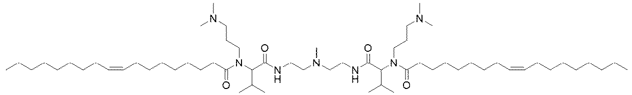 |
| A1I4R2C18-2 | 3.18E+03 | 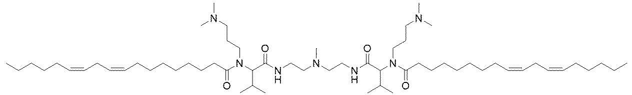 |
| A1I4R13C18 | 5.76E+02 | 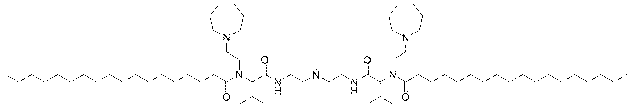 |
| A1I4R13C18-1 | 2.67E+03 | 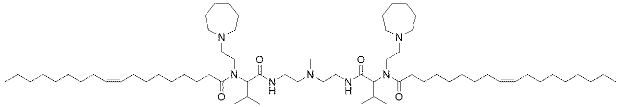 |
| A1I4R13C18-2 | 2.85E+04 | 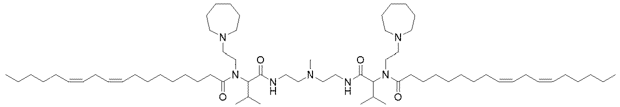 |
| A1I4R21C18 | 3.46E+04 | 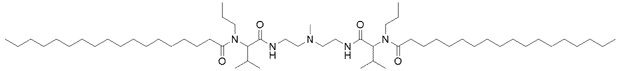 |
| A1I4R21C18-1 | 2.76E+05 | 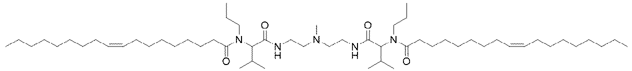 |
| A1I4R21C18-2 | 6.62E+05 | 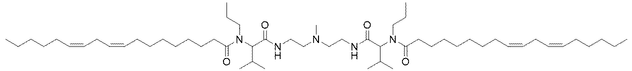 |
| A1I4R22C18 | 1.10E+05 | 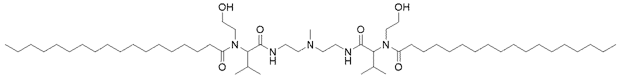 |
| A1I4R22C18-1 | 3.18E+05 | 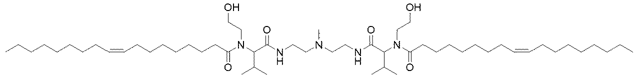 |
| A1I4R22C18-2 | 1.47E+06 | 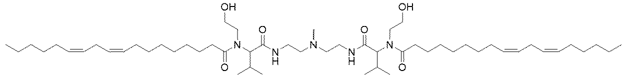 |
| A1I4R23C18 | 1.51E+05 | 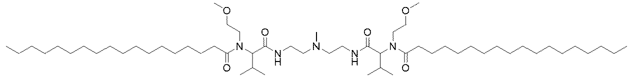 |
| A1I4R23C18-1 | 3.11E+05 | 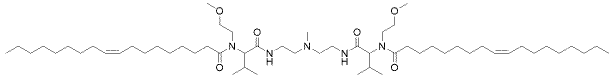 |
| A1I4R23C18-2 | 9.64E+05 | 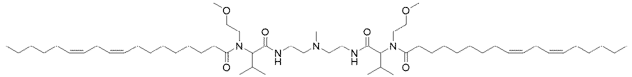 |
| A4I4R2C18 | 2.24E+03 | 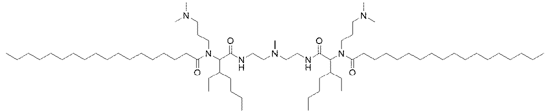 |
| A4I4R2C18-1 | 1.13E+03 | 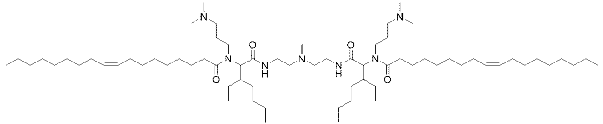 |
| A4I4R2C18-2 | 1.86E+04 | 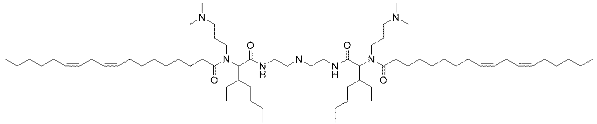 |
| A4I4R13C18 | 2.79E+03 | 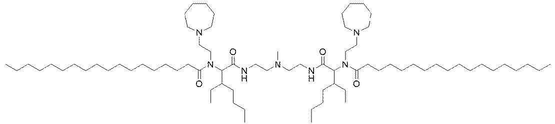 |
| A4I4R13C18-1 | 1.59E+03 | 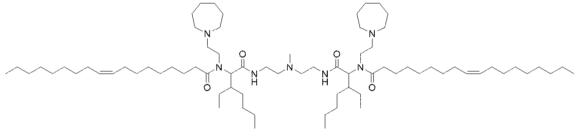 |
| A4I4R13C18-2 | 1.19E+04 | 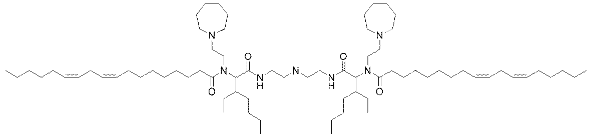 |
| A4I4R21C18 | 2.09E+04 | 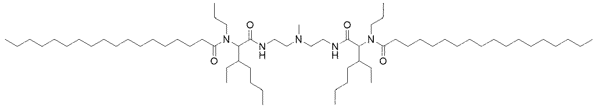 |
| A4I4R21C18-1 | 3.02E+04 | 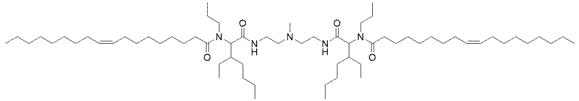 |
| A4I4R21C18-2 | 1.91E+04 | 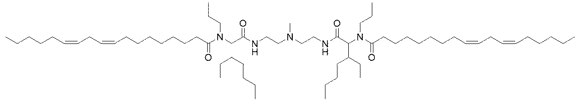 |
| A4I4R22C18 | 5.51E+05 | 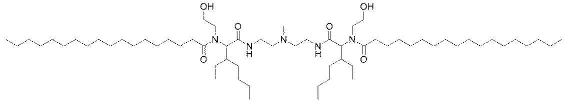 |
| A4I4R22C18-1 | 6.82E+05 | 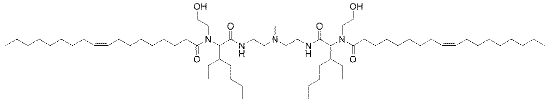 |
| A4I4R22C18-2 | 2.61E+05 | 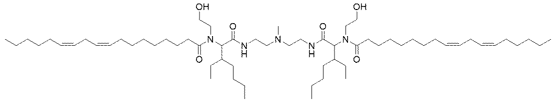 |
| A4I4R23C18 | 2.16E+05 | 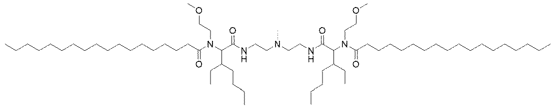 |
| A4I4R23C18-1 | 6.08E+04 | 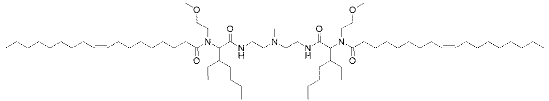 |
| A4I4R23C18-2 | 4.96E+05 | 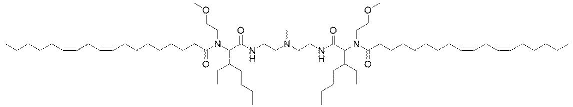 |
| A21I4R2C18 | 1.04E+03 | 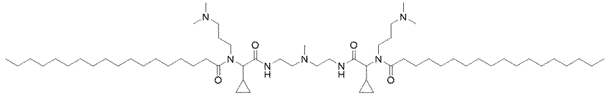 |
| A21I4R2C18-1 | 8.38E+02 | 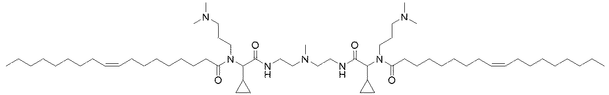 |
| A21I4R2C18-2 | 1.83E+03 | 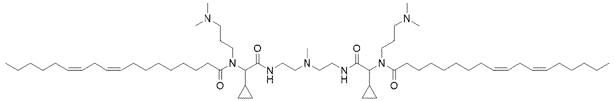 |
| A21I4R13C18 | 1.12E+03 | 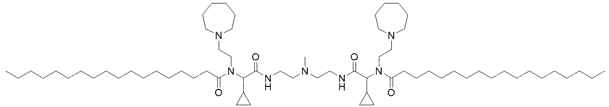 |
| A21I4R13C18-1 | 9.59E+02 | 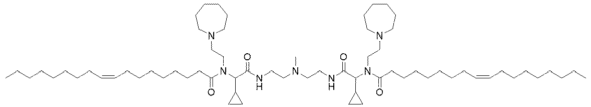 |
| A21I4R13C18-2 | 8.67E+02 | 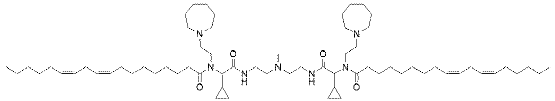 |
| A21I4R21C18 | 5.31E+03 | 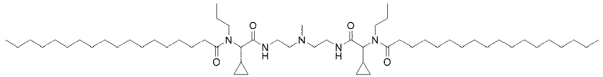 |
| A21I4R21C18-1 | 8.50E+03 | 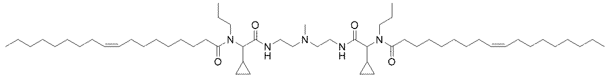 |
| A21I4R21C18-2 | 1.39E+03 | 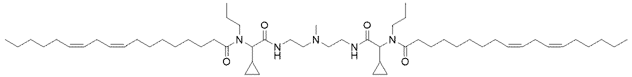 |
| A21I4R22C18 | 1.28E+05 | 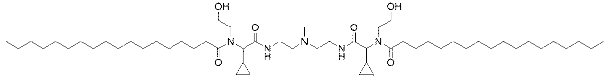 |
| A21I4R22C18-1 | 1.76E+04 | 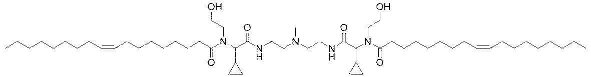 |
| A21I4R22C18-2 | 1.26E+03 | 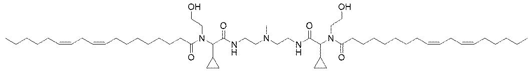 |
| A21I4R23C18 | 2.65E+04 | 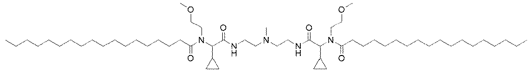 |
| A21I4R23C18-1 | 2.28E+04 | 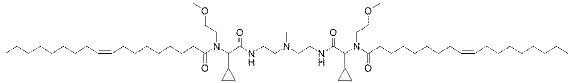 |
| A21I4R23C18-2 | 6.97E+04 | 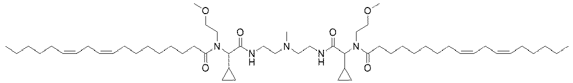 |
| A23I4R2C18 | 5.71E+02 | 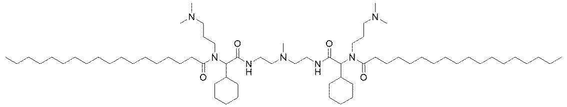 |
| A23I4R2C18-1 | 1.78E+03 | 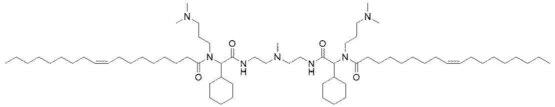 |
| A23I4R2C18-2 | 2.18E+03 | 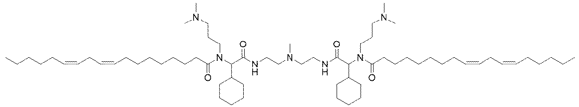 |
| A23I4R13C18 | 7.89E+02 | 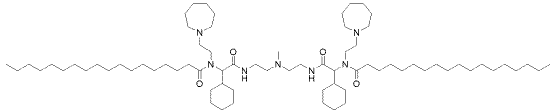 |
| A23I4R13C18-1 | 6.61E+02 | 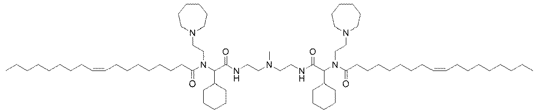 |
| A23I4R13C18-2 | 9.46E+03 | 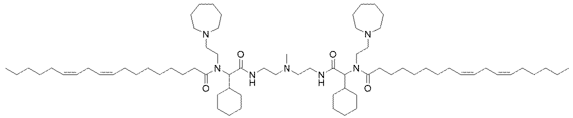 |
| A23I4R21C18 | 5.43E+03 | 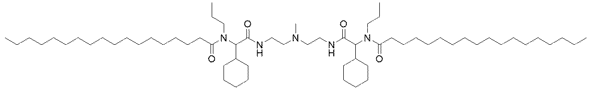 |
| A23I4R21C18-1 | 9.63E+04 | 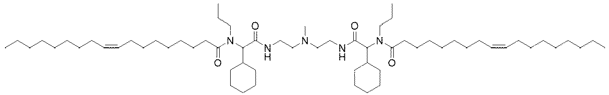 |
| A23I4R21C18-2 | 6.07E+04 | 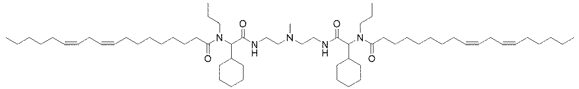 |
| A23I4R22C18 | 4.61E+05 | 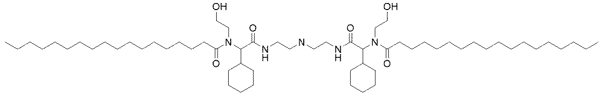 |
| A23I4R22C18-1 | 6.57E+05 | 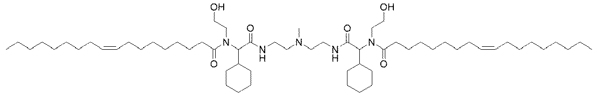 |
| A23I4R22C18-2 | 6.10E+05 | 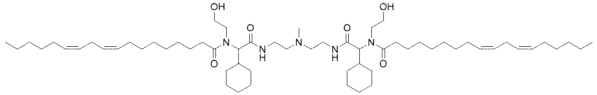 |
| A23I4R23C18 | 1.49E+05 | 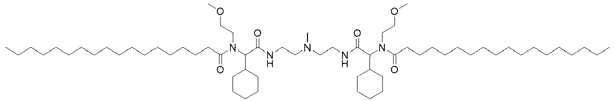 |
| A23I4R23C18-1 | 2.96E+05 | 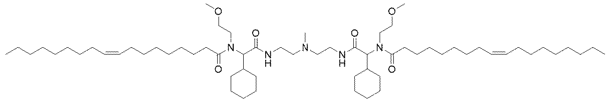 |
| A23I4R23C18-2 | 1.04E+05 | 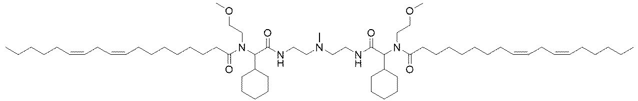 |
| A1I4R22C12 | 3.90E+03 | 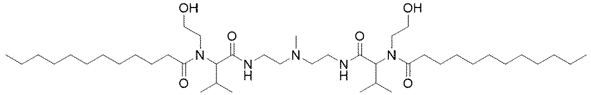 |
| A1I4R22C14 | 2.30E+03 | 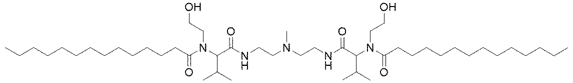 |
| A1I4R22C16 | 2.30E+03 | 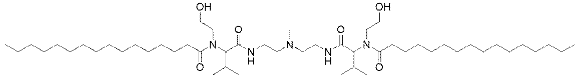 |
| A1I4R22S11 | 1.00E+03 | 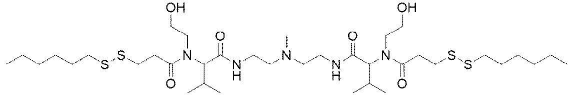 |
| A1I4R22S13 | 5.90E+03 | 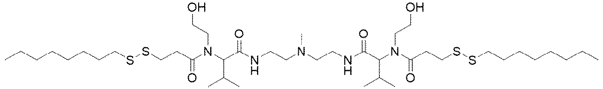 |
| A1I4R22S15 | 4.10E+03 | 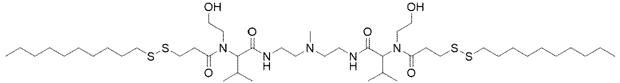 |
| A1I4R22S17 | 1.80E+04 | 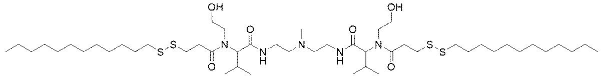 |
| A1I4R22S19 | 2.50E+04 | 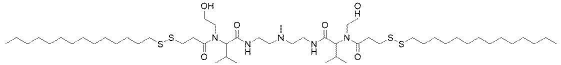 |

**Table 4 In vivo expression levels of mRNA-loaded nanoparticles prepared by the compounds of Example 3 after intravenous injection**

| Name of the cationic lipid | Fluorescence intensity of | Structural formula |
|---|---|---|
| analogue | spleen | |
| A1I5R22C8 | 1.50E+05 | 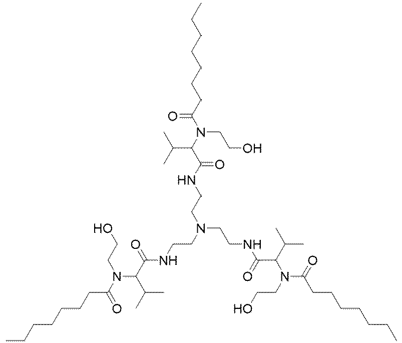 |
| A1I5R22C10 | 3.00E+05 | 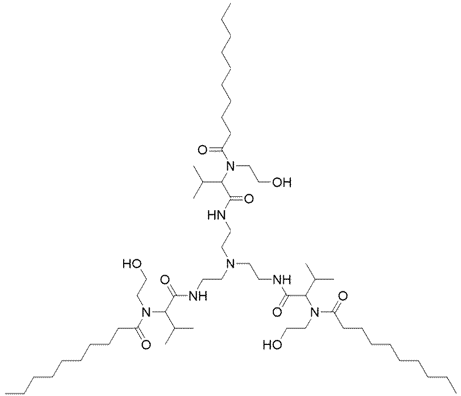 |
| A1I5R22C12 | 2.96E+06 | 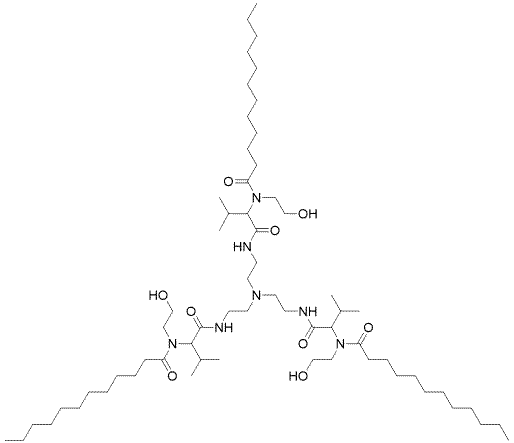 |
| A1I5R22C14 | 1.20E+06 | 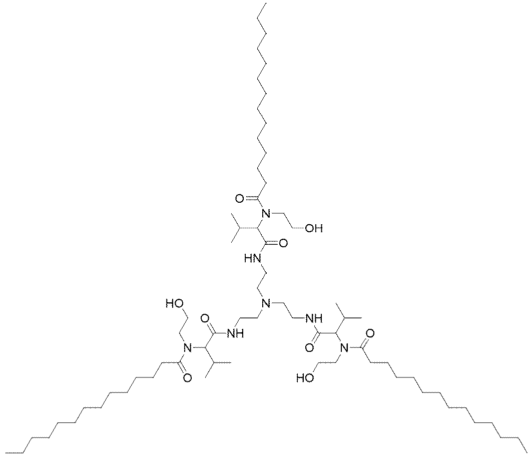 |
| A1I5R22C16 | 4.10E+05 | 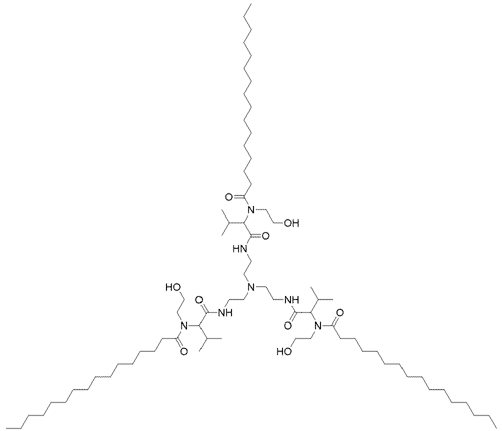 |
| A1I5R22C18 | 1.10E+05 | 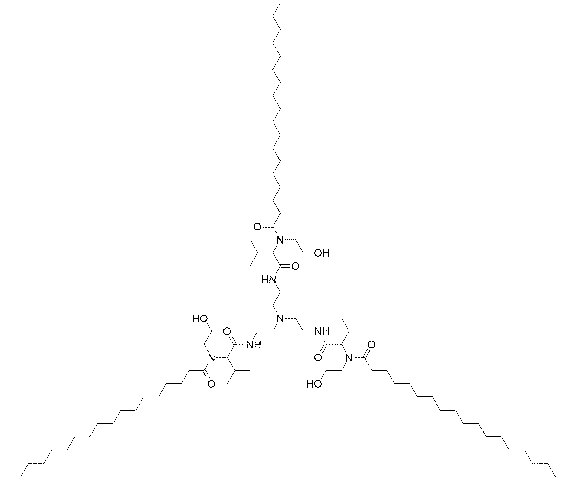 |
| A1I5R22C18-2 | 6.90E+05 | 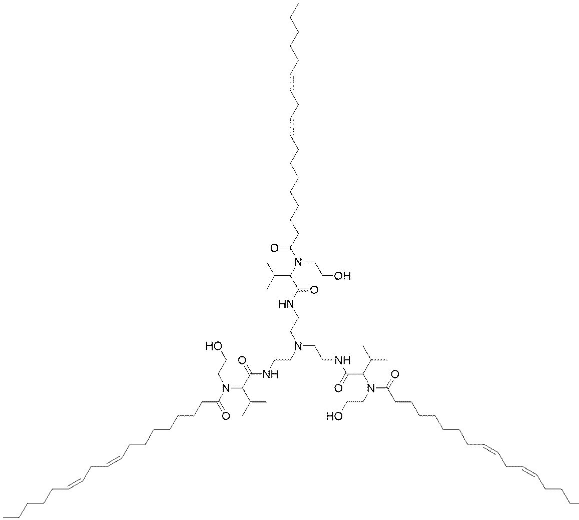 |
| A1I5R22S11 | 1.30E+05 | 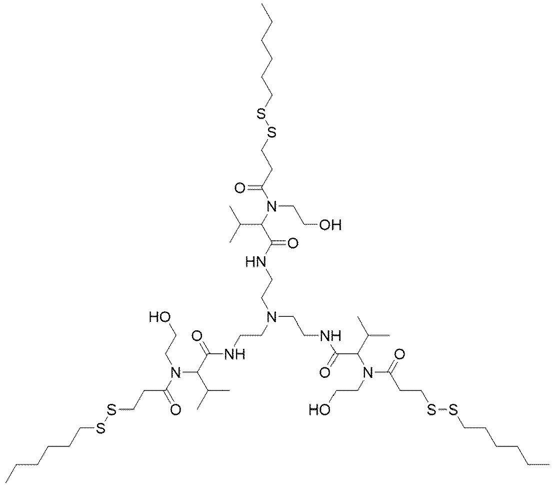 |
| A1I5R22S13 | 1.60E+06 | 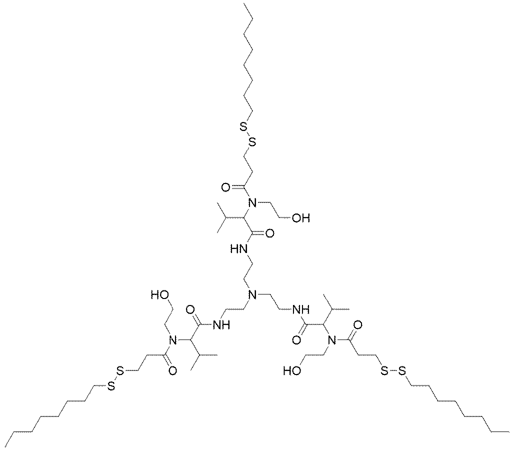 |
| A1I5R22S15 | 3.40E+06 | 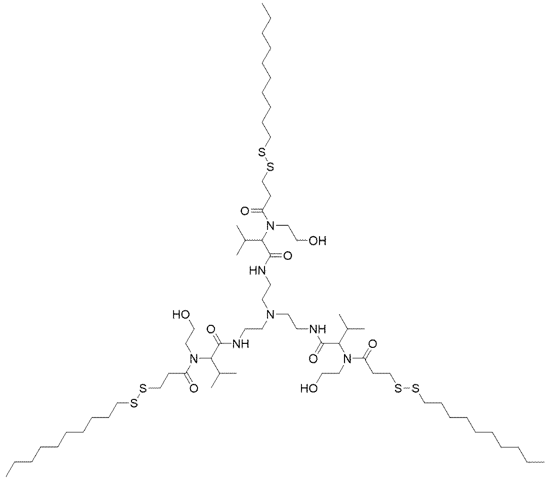 |
| A1I5R22S17 | 6.40E+05 | 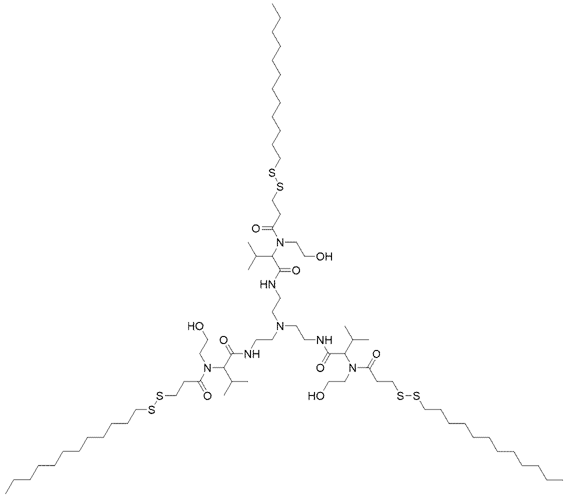 |
| A1I5R22S19 | 1.80E+06 | 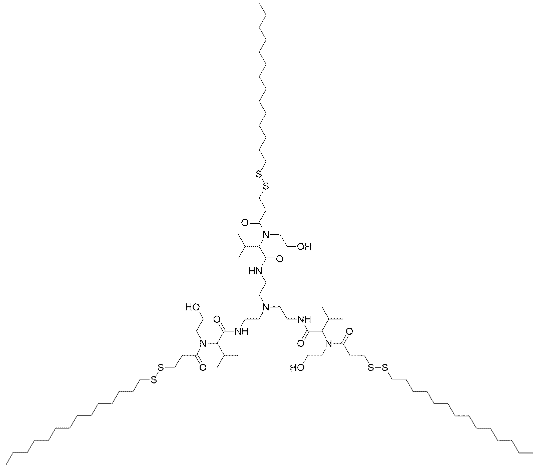 |
| A1I5R26S15 | 1.30E+05 | 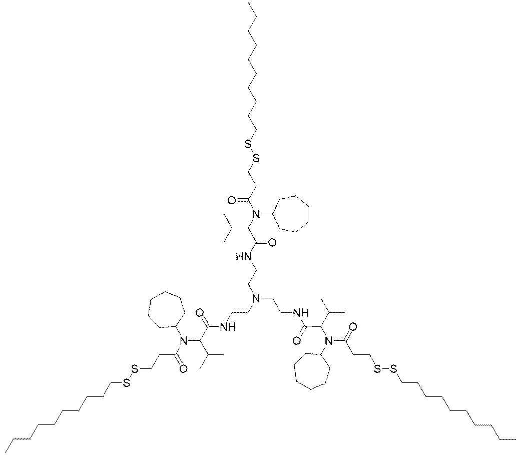 |
| A1I5R27S15 | 7.96E+05 | 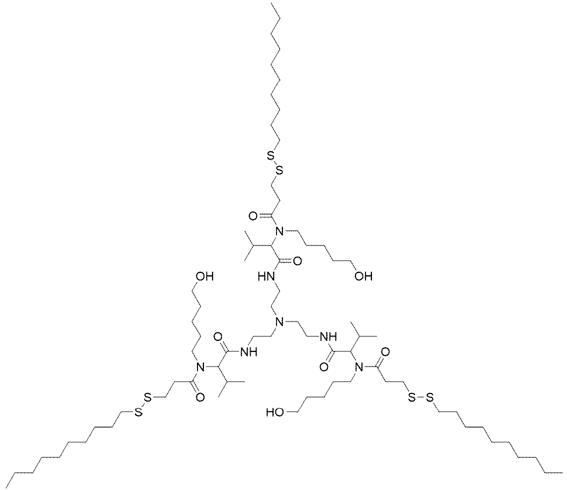 |
| A1I5R28S15 | 3.31E+06 | 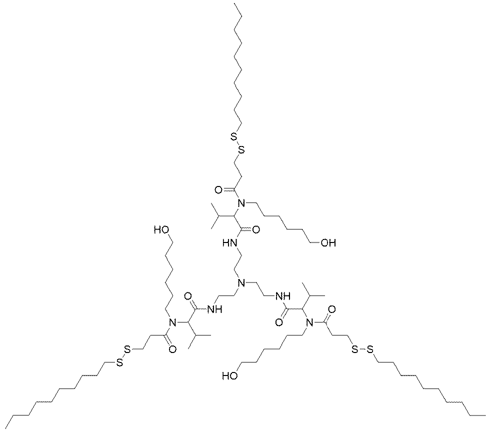 |
| A1I5R29S15 | 3.13E+05 | 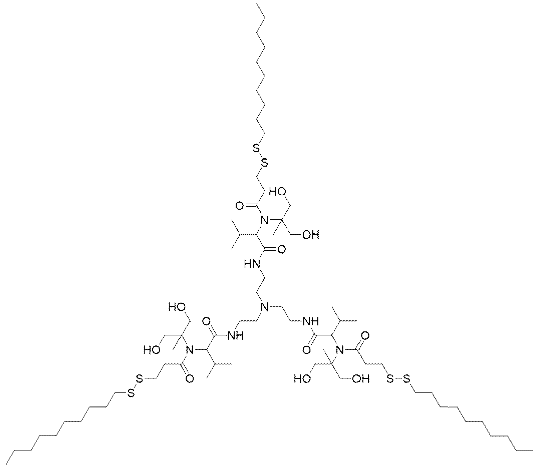 |
| A1I5R30S15 | 6.31E+04 | 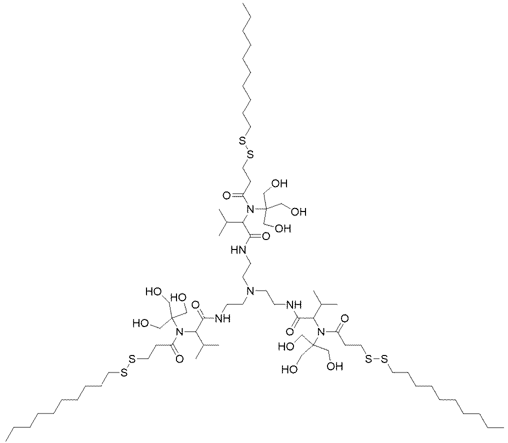 |
| A1I5R31S15 | 6.13E+05 | 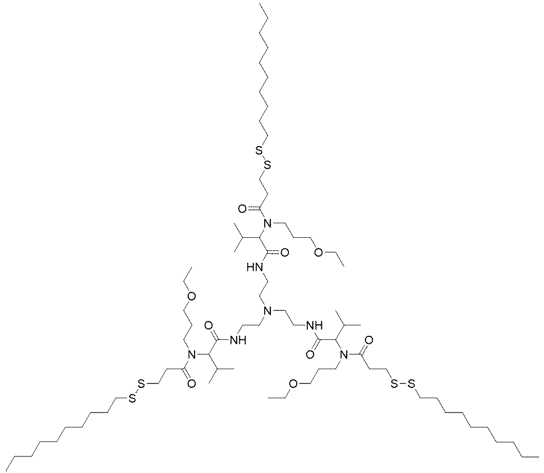 |
| A1I5R32S15 | 2.48E+05 | 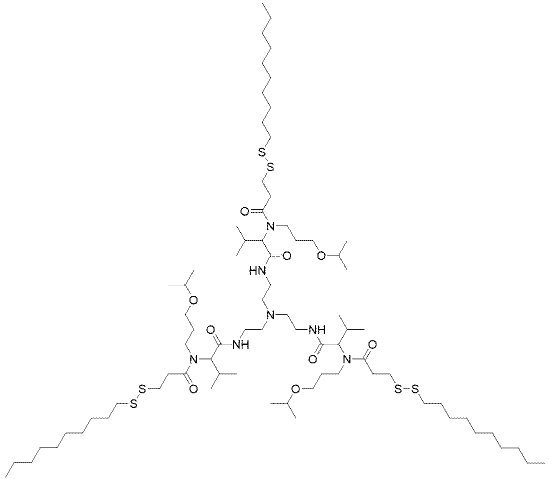 |
| A1I5R33S15 | 2.33E+05 | 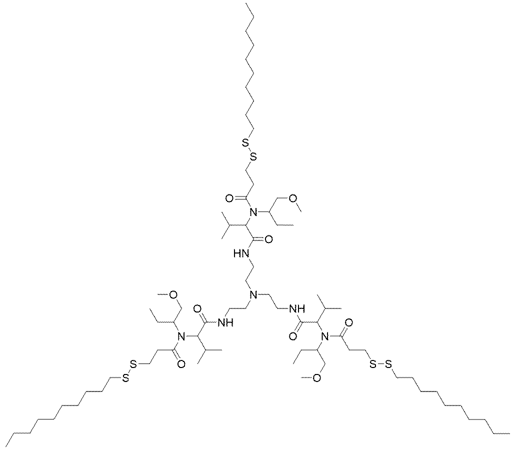 |
| A1I5R34S15 | 2.84E+05 | 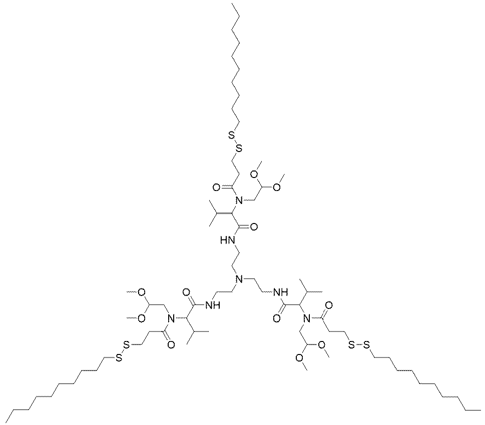 |
| A1I5R35S15 | 3.21E+05 | 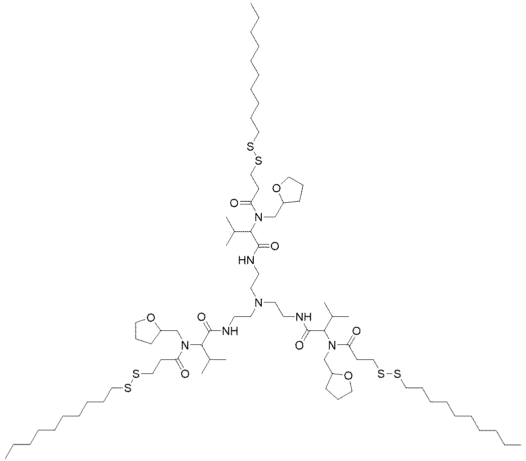 |
| A1I5R36S15 | 3.27E+06 | 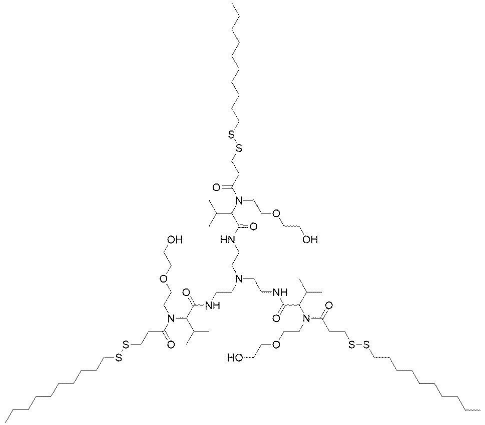 |
| A1I5R37S15 | 1.61E+05 | 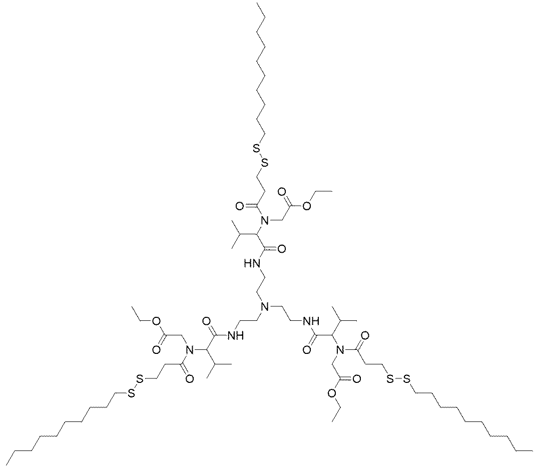 |
| A1I5R38S15 | 1.14E+05 | 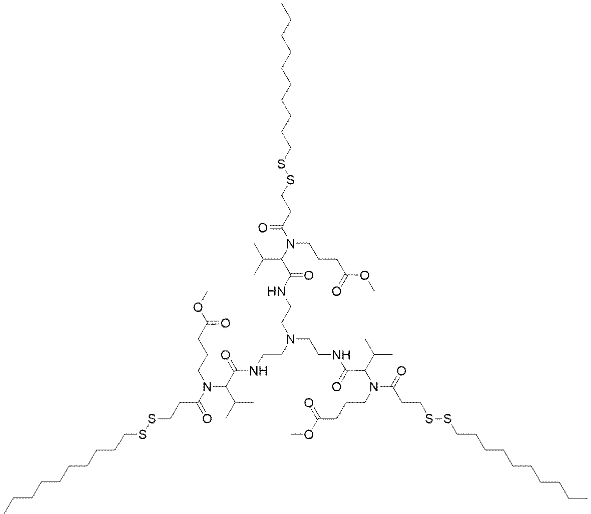 |
| A1I5R39S15 | 3.88E+04 | 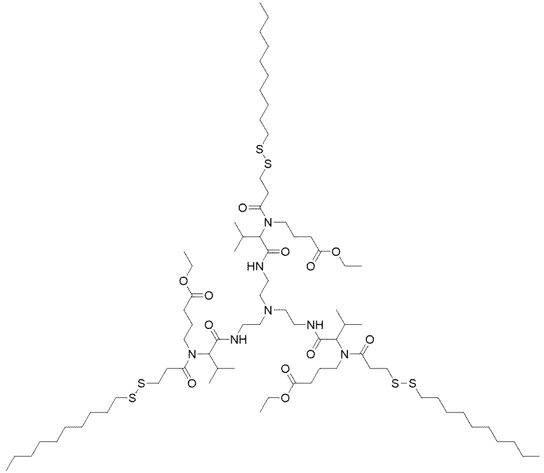 |
| A1I5R40S15 | 1.39E+04 | 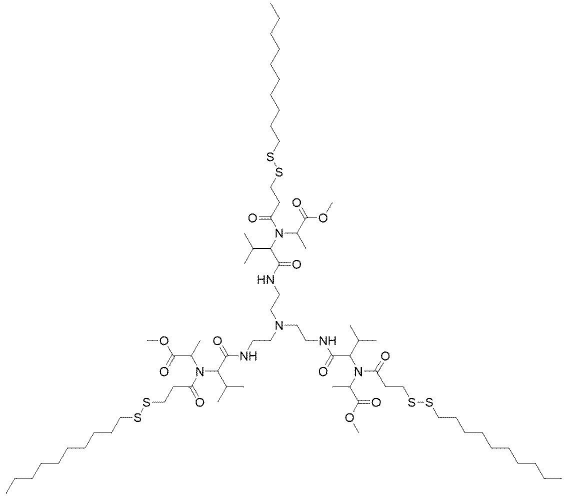 |
| A1I5R41S15 | 4.22E+05 | 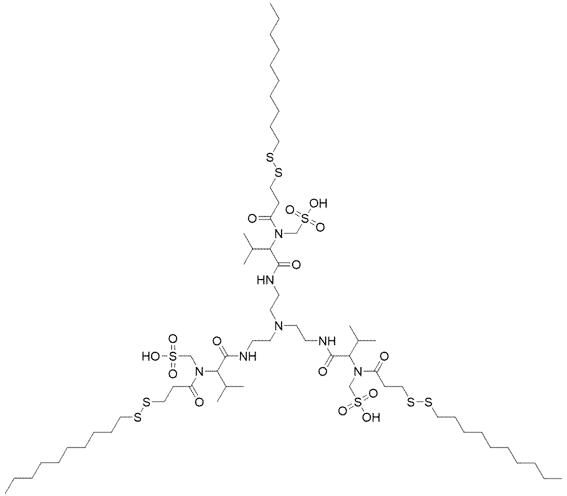 |
| A1I5R50C21 | 3.60E+06 | 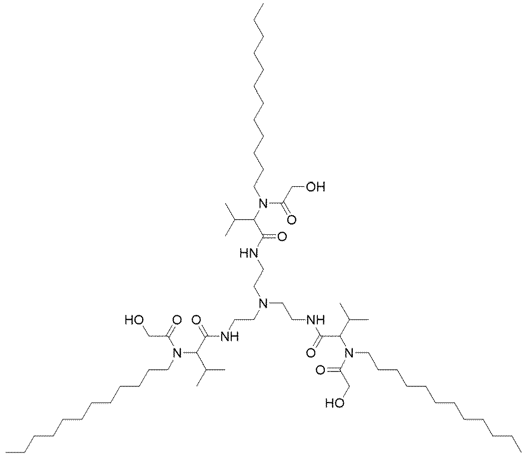 |

**Table 5 In vivo expression levels of mRNA-loaded nanoparticles prepared by the compounds of Example 4 after intravenous injection**

| Name of the cationic lipid analogue | Fluorescence intensity of spleen | Structural formula |
|---|---|---|
| A1I31R46C18-2 | 2.60E+05 | 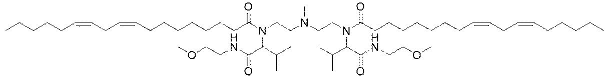 |
| A1I32R46C18-2 | 4.50E+04 | 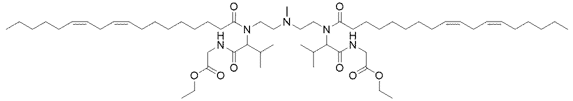 |
| A1I12R51C8 | 1.60E+03 | 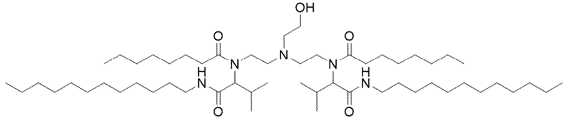 |
| A1I10R51C18-2 | 4.00E+04 | 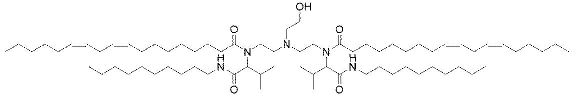 |

**Table 6 In vivo expression levels of mRNA-loaded nanoparticles prepared by the compounds of Example 5 after intravenous injection**

| Name of the cationic lipid analogue | Fluorescence intensity of spleen | Structural formula |
|---|---|---|
| A1I12R47C21 | 3.10E+05 | 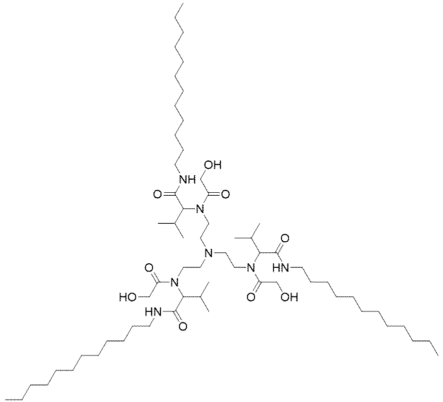 |
| A1I31R47C18-2 | 6.90E+04 | 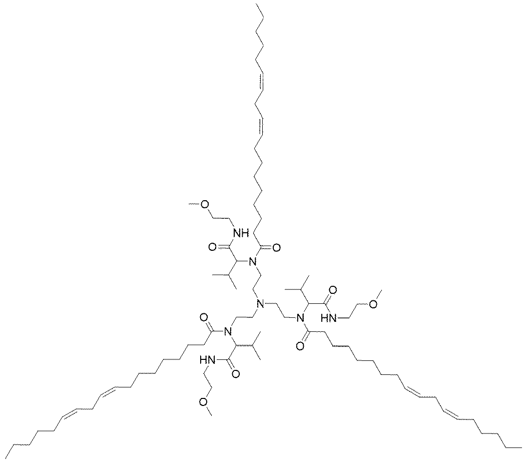 |
| A1I32R47C18-2 | 8.50E+03 | 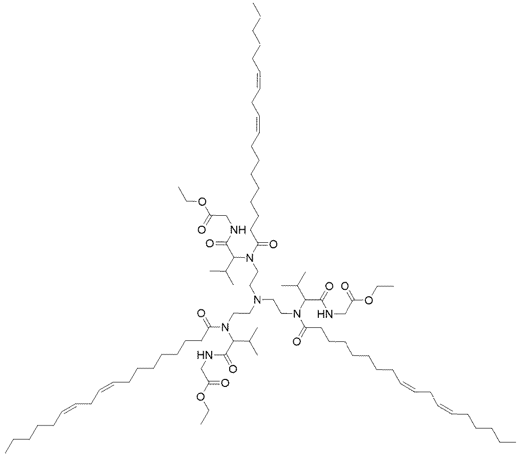 |

### Example 11: An animal experiment for detecting the in vivo expression level of mRNA delivered by intramuscular injection

Luciferase-mRNA was used as an mRNA model; and the cationic lipid analogues of Examples 1-5 were used to prepare drug-loaded nanoparticles according to the method of Example 6.

Detailed operating processes were as follows:
100 µL of nanoparticles containing 5 µg Luciferase-mRNA or the drug-loaded nanoparticles prepared above were injected into the leg muscles of 6-8 week-old C57BL/6 mice, and 200 µL of 1× PBS solution containing 3 mg D-Luciferin potassium salt was intraperitoneally injected into the mice 6 hours later. After waiting for 10 minutes, an in vivo expression level of the mRNA was evaluated by bioluminescence imaging.

**Table 7 In vivo expression levels of mRNA-loaded nanoparticles prepared by the compounds of Example 1 after intramuscular injection**

| Name of the cationic lipid analogue | Local fluorescence intensity | Structural formula |
|---|---|---|
| A1I18R2C18 | 1.36E+05 | 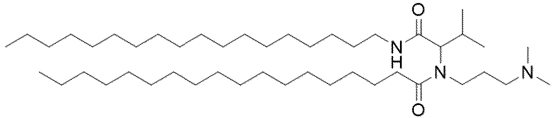 |
| A1I18R2C18-2 | 7.88E+04 | 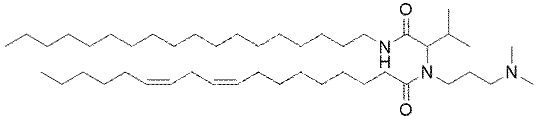 |
| A1I18R5C18 | 5.25E+04 | 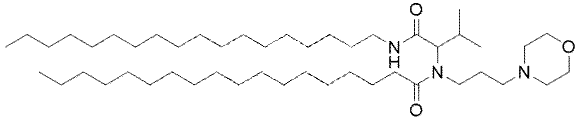 |
| A1I18R5C18-2 | 8.15E+04 | 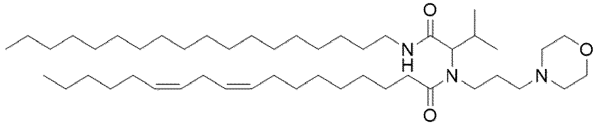 |
| A1I18R12C18 | 4.37E+04 | 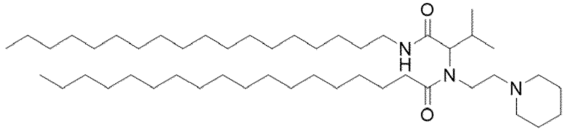 |
| A1I18R12C18-2 | 3.38E+06 | 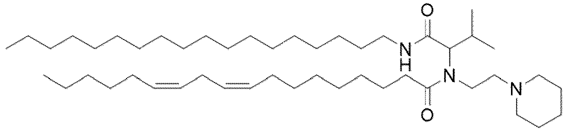 |
| A4I18R2C18 | 9.04E+05 | 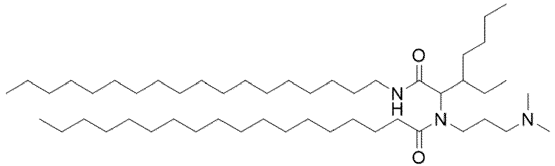 |
| A4I18R2C18-2 | 2.97E+07 | 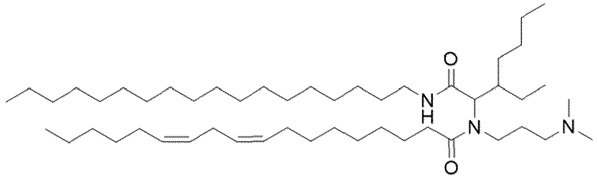 |
| A4I18R5C18 | 4.33E+05 | 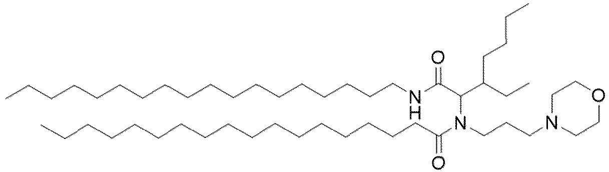 |
| A4I18R5C18-2 | 4.99E+05 | 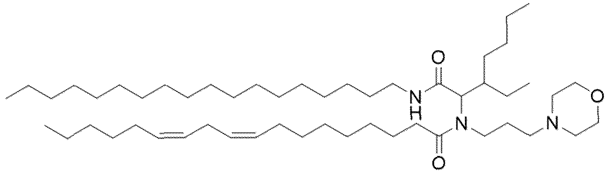 |
| A4I18R12C18 | 1.75E+05 | 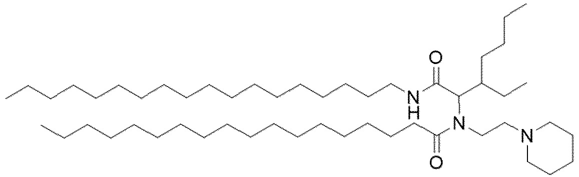 |
| A4I18R12C18-2 | 2.60E+05 | 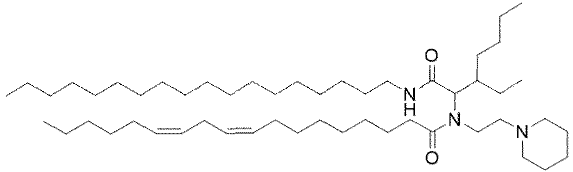 |
| A4I18R1C18-1 | 1.54E+07 | 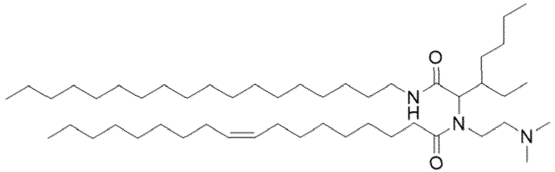 |
| A4I18R2C18-1 | 1.32E+07 | 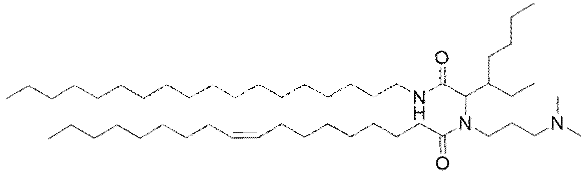 |
| A4I18R3C18-1 | 1.77E+07 | 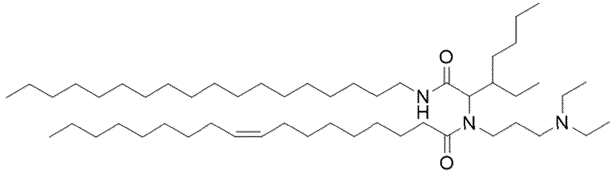 |
| A4I18R1C18-2 | 2.11E+07 | 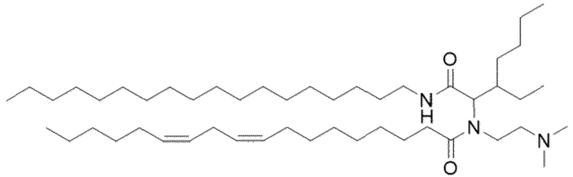 |
| A4I18R3C18-2 | 3.08E+07 | 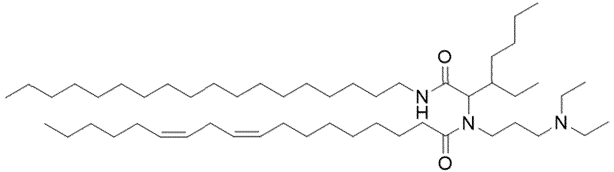 |
| A23I18R2C18 | 4.83E+04 | 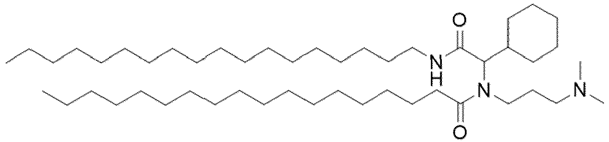 |
| A23I18R2C18-2 | 7.16E+05 | 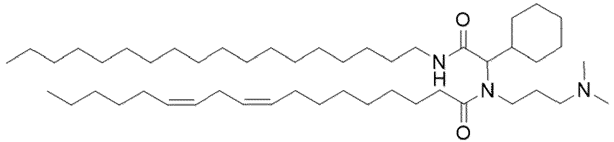 |
| A23I18R5C18 | 9.13E+04 | 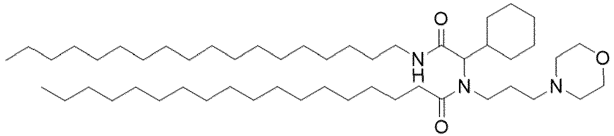 |
| A23I18R5C18-2 | 4.37E+05 | 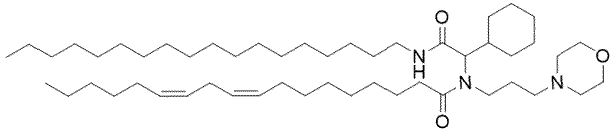 |
| A23I18R12C18 | 1.03E+06 | 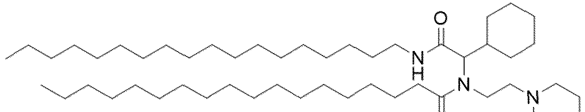 |
| A23I18R12C18-2 | 2.01E+06 | 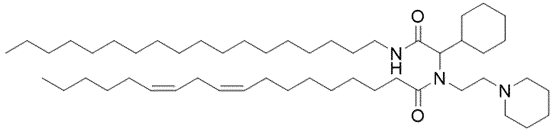 |

**Table 8 In vivo expression levels of mRNA-loaded nanoparticles prepared by the compounds of Example 2 after intramuscular injection**

| Name of the cationic lipid analogue | Local fluorescen ce intensity | Structural formula |
|---|---|---|
| A1I4R21C18-1 | 2.26E+07 | 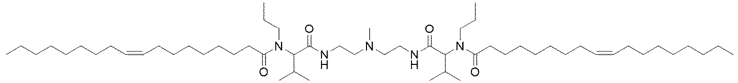 |
| A1I4R21C18-2 | 3.17E+05 | 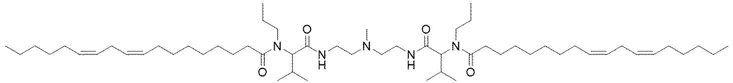 |
| AI41R22C18 | 3.41E+04 | 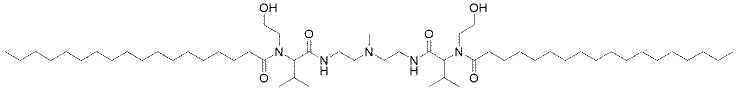 |
| A1I4R22C18-1 | 2.86E+04 | 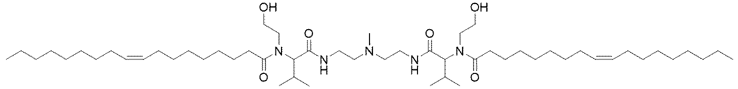 |
| A1I4R22C18-2 | 2.69E+05 | 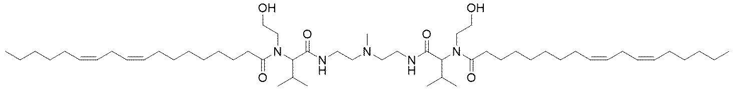 |
| AI41R23C18 | 7.53E+04 | 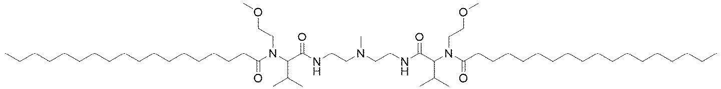 |
| A1I4R23C18-1 | 1.77E+05 | 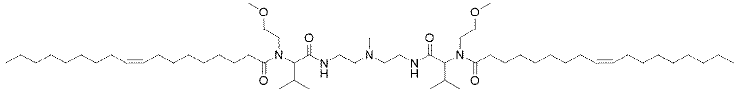 |
| A1I4R23C18-2 | 8.72E+05 | 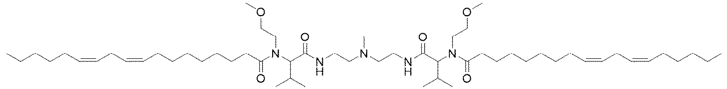 |
| A4I4R22C18 | 5.53E+05 | 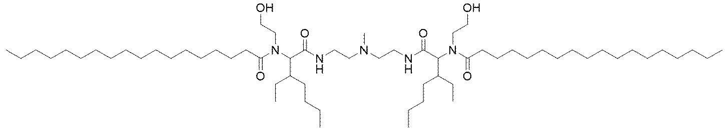 |
| A4I4R22C18-1 | 5.47E+05 | 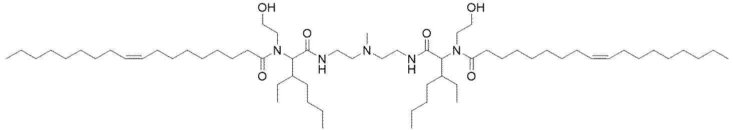 |
| A4I4R22C18-2 | 4.66E+05 | 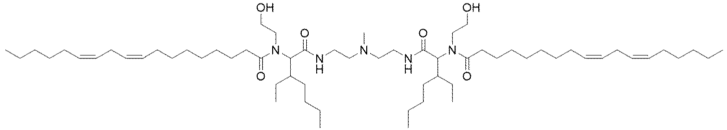 |
| A4I4R23C18 | 6.36E+04 | 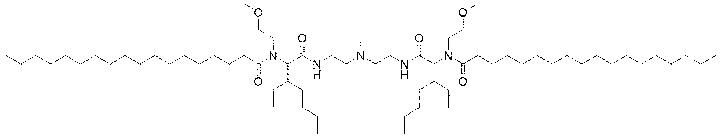 |
| A4I4R23C18-2 | 1.93E+04 | 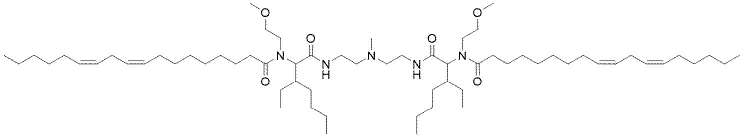 |
| A21I4R22C18 | 1.53E+05 | 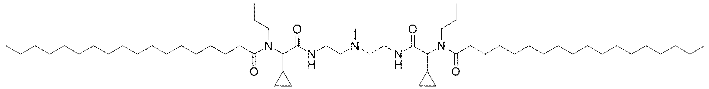 |
| A23I4R22C18 | 3.41E+04 | 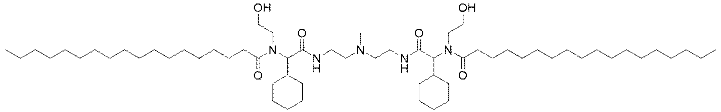 |
| A23I4R22C18-1 | 1.41E+05 | 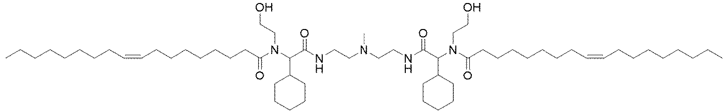 |
| A23I4R22C18-2 | 9.09E+05 | 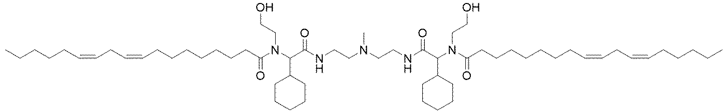 |
| A23I4R23C18 | 5.32E+05 | 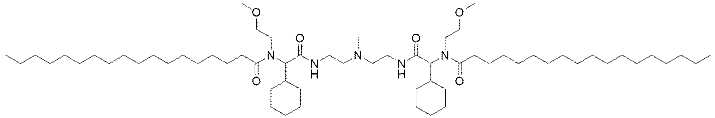 |
| A23I4R23C18-1 | 5.87E+06 | 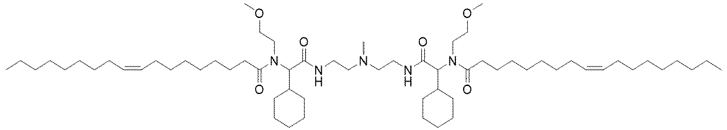 |
| A1I4R27C18-2 | 1.67E+04 | 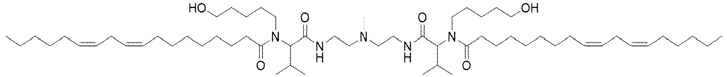 |
| A1I4R28C18-2 | 1.72E+06 | 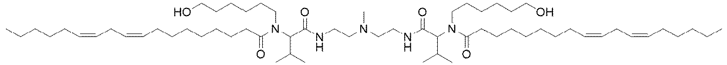 |
| A1I4R29C18-2 | 2.99E+04 | 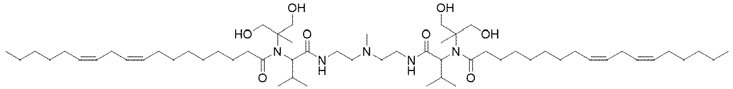 |
| A1I4R30C18-2 | 2.05E+04 | 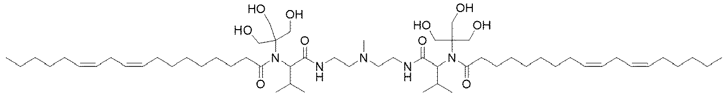 |
| A1I4R31C18-2 | 5.21E+04 | 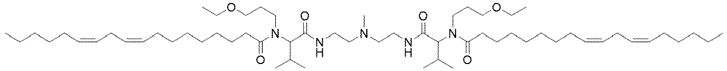 |
| A1I4R32C18-2 | 2.57E+04 | 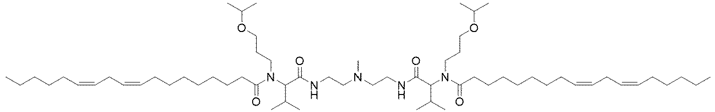 |
| A1I4R33C18-2 | 1.02E+05 | 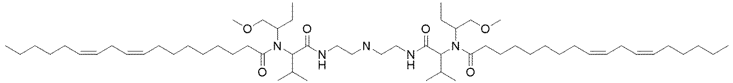 |
| A1I4R34C18-2 | 5.58E+05 | 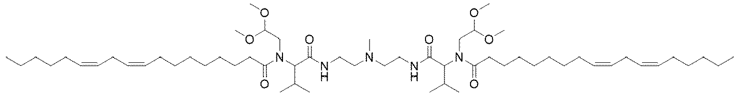 |
| A1I4R35C18-2 | 3.38E+05 | 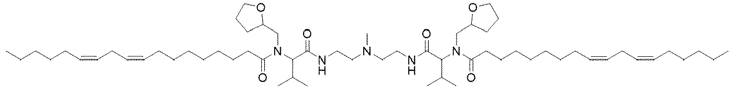 |
| A1I4R36C18-2 | 5.68E+04 | 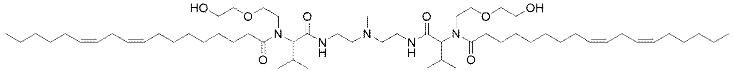 |
| A1I4R42C18-2 | 7.04E+04 | 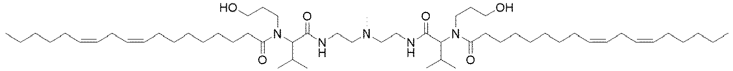 |
| A1I4R43C18-2 | 6.57E+04 | 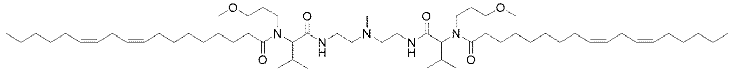 |
| A1I4R48C18-2 | 8.55E+04 | 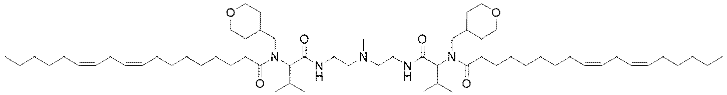 |

**Table 9 In vivo expression levels of mRNA-loaded nanoparticles prepared by the compounds of Example 3 after intramuscular injection**

| Name of the cationic lipid analogues | Local fluorescence intensity | Structural formula |
|---|---|---|
| A1I5R22C14 | 1.08E+05 | 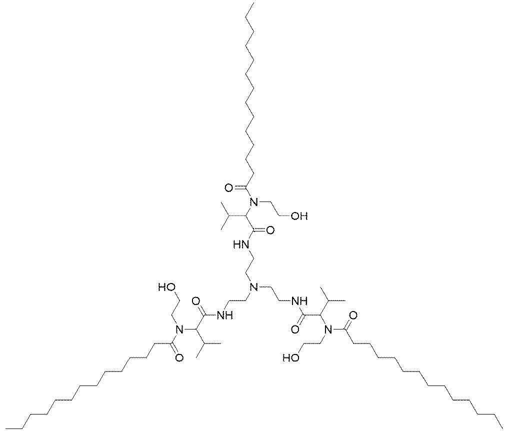 |
| A1I5R22S13 | 5.74E+05 | 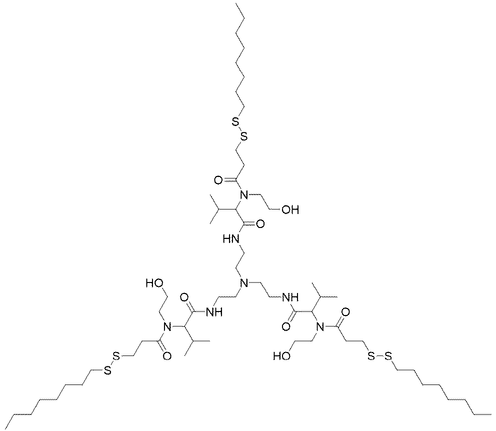 |
| A1I5R22S15 | 1.50E+06 | 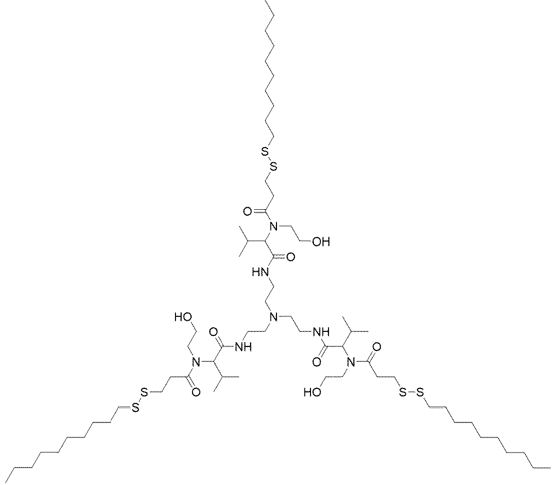 |
| A1I5R26S15 | 9.90E+05 | 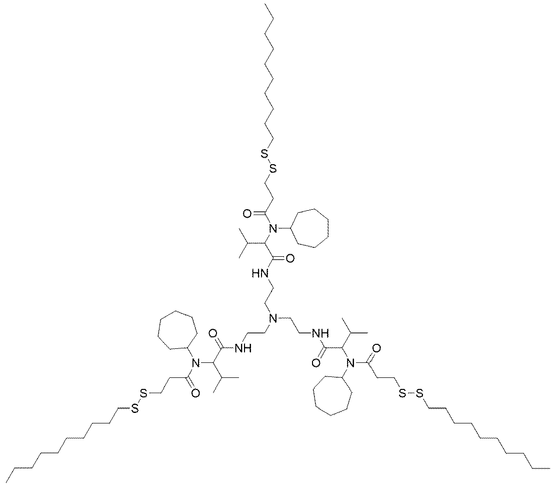 |
| A1I5R27S15 | 3.57E+05 | 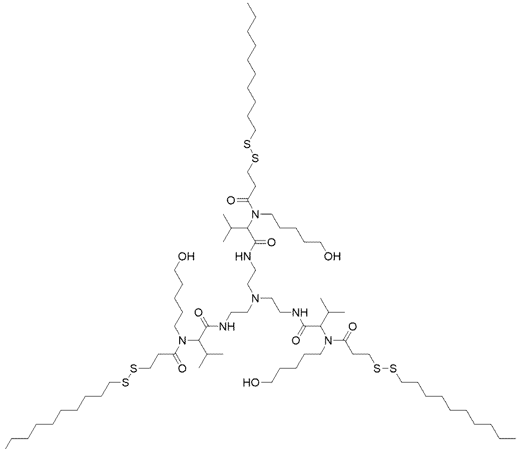 |
| A1I5R28S15 | 6.01E+05 | 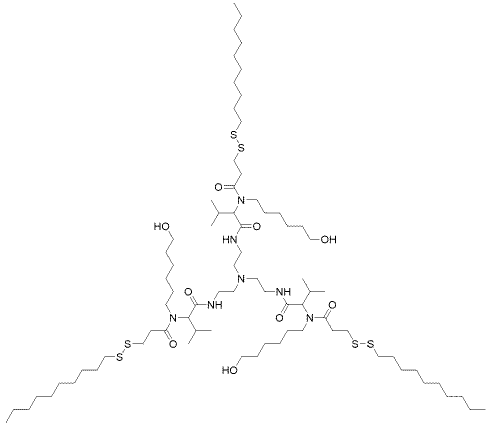 |
| A1I5R29S15 | 7.63E+04 | 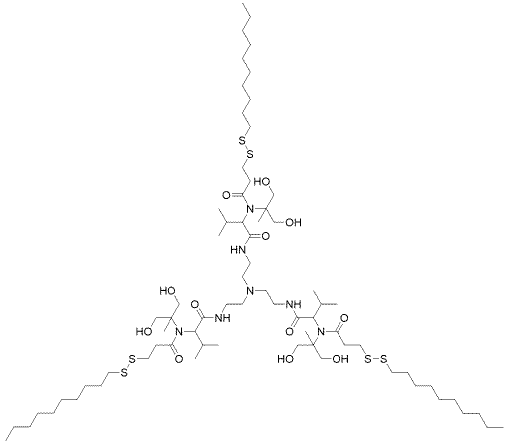 |
| A1I5R30S15 | 7.77E+04 | 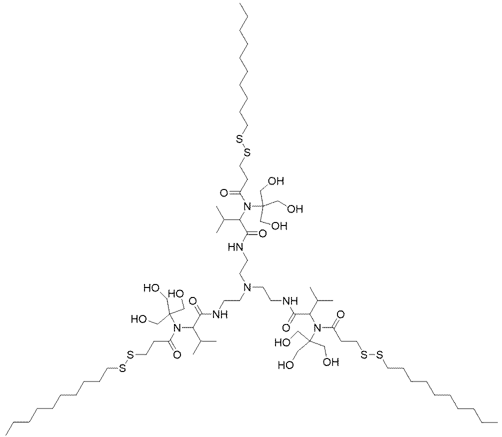 |
| A1I5R31S15 | 2.94E+06 | 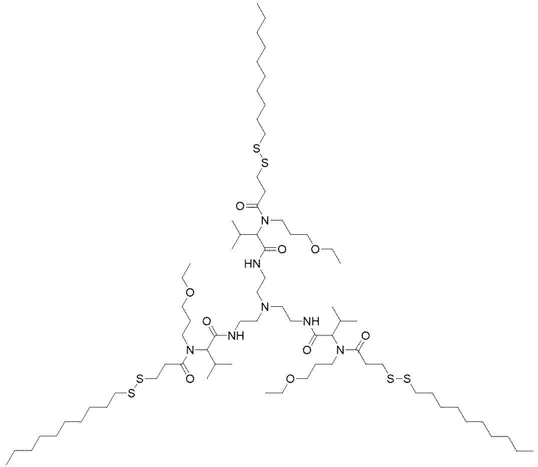 |
| A1I5R32S15 | 1.18E+06 | 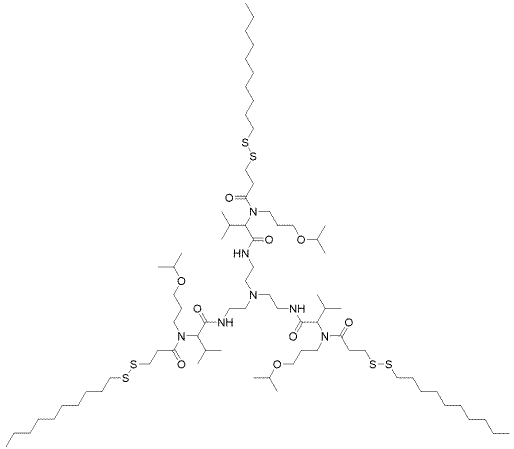 |
| A1I5R33S15 | 1.52E+06 | 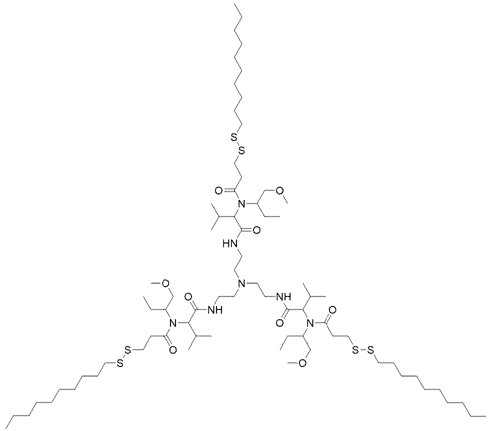 |
| A1I5R34S15 | 1.24E+06 | 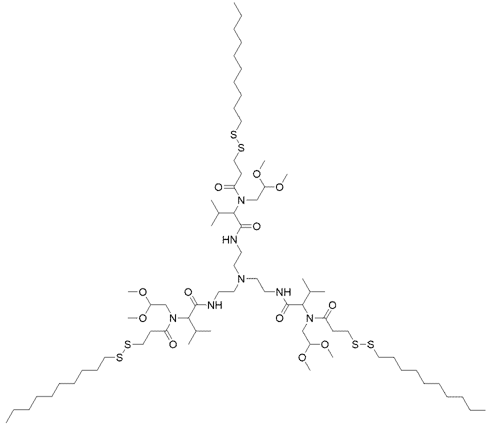 |
| A1I5R35S15 | 2.62E+06 | 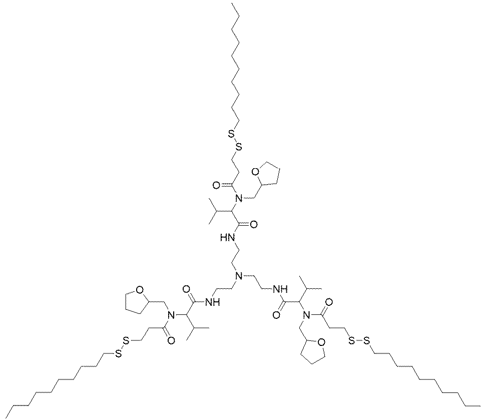 |
| A1I5R36S15 | 8.27E+04 | 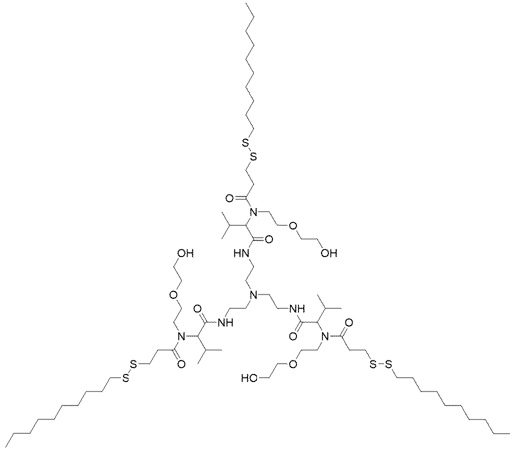 |
| A1I5R37S15 | 1.26E+06 | 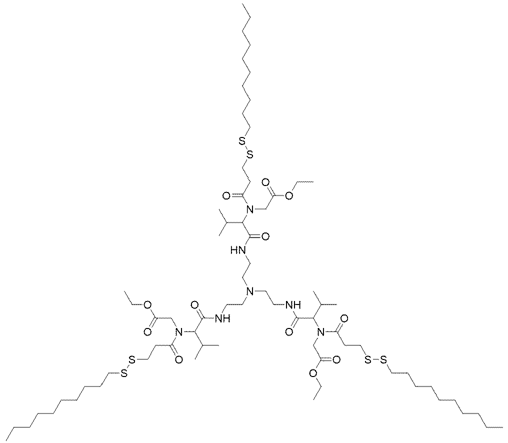 |
| A1I5R38S15 | 4.72E+04 | 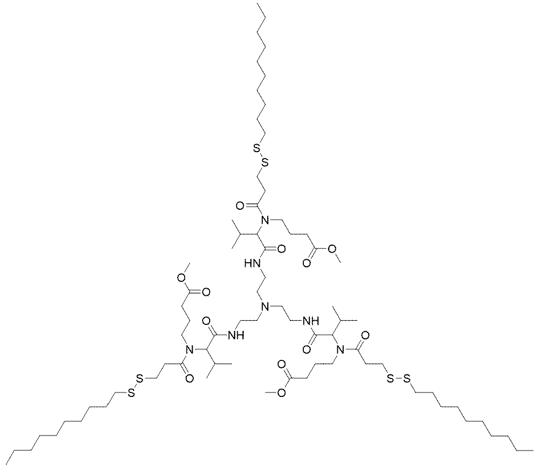 |
| A1I5R39S15 | 2.15E+04 | 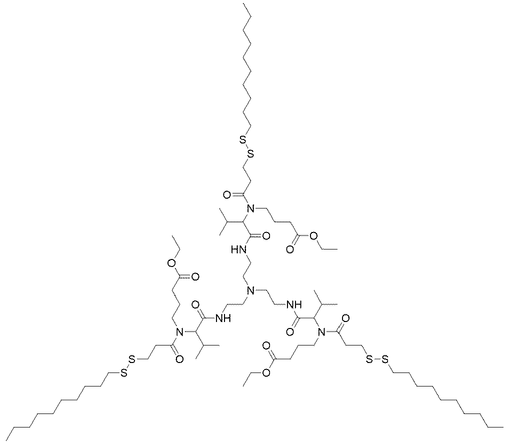 |
| A1I5R40S15 | 3.86E+05 | 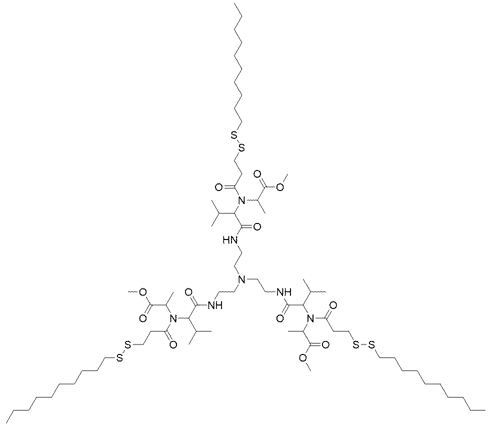 |
| A1I5R41S15 | 1.81E+05 | 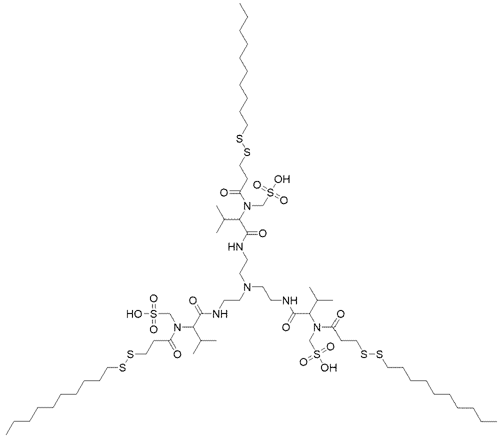 |

**Table 10 In vivo expression levels of mRNA-loaded nanoparticles prepared by the compounds of Example 4 after intramuscular injection**

| Name of the cationic lipid analogue | Local fluorescence intensity | Structural formula |
|---|---|---|
| A1I12R51C8 | 1.28E+05 | 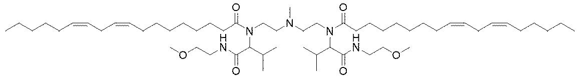 |
| A1I10R51C18-2 | 2.39E+04 | 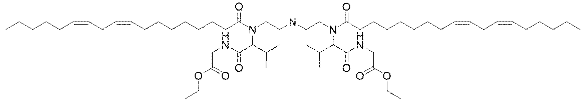 |
| A1I31R46C18-2 | 2.75E+06 | 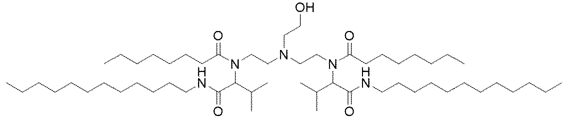 |
| A1I32R46C18-2 | 1.43E+06 | 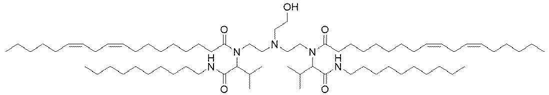 |

**Table 11 In vivo expression levels of mRNA-loaded nanoparticles prepared by the compounds of Example 5 after intramuscular injection**

| Name of the cationic lipid analogue | Local fluorescence intensity | Structural formula |
|---|---|---|
| A1I12R47C21 | 2.22E+05 | 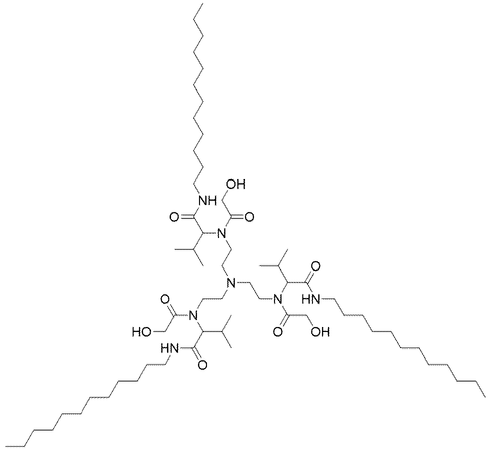 |
| A1I31R47C18-2 | 2.24E+05 | 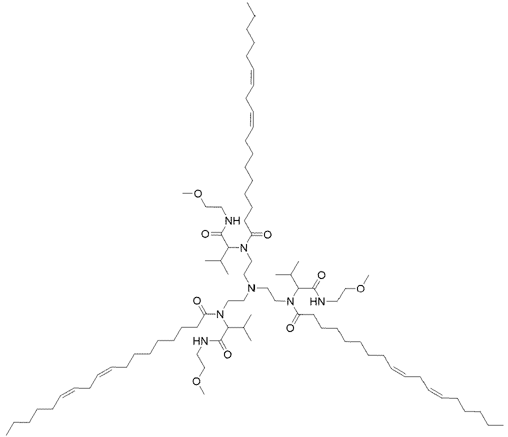 |
| A1I32R47C18-2 | 3.68E+04 | 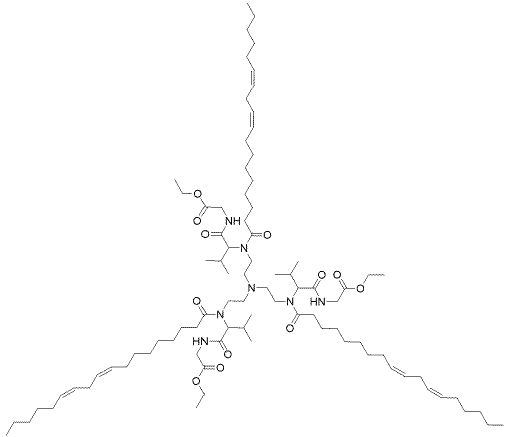 |

### Example 12: An animal experiment for detecting the in vivo expression level of mRNA delivered by intraperitoneal injection

Luciferase-mRNA was used as an mRNA model; and the cationic lipid analogue A1I4R22C18-2 of Example 2 was used to prepare drug-loaded nanoparticles according to the method of Example 6.

Detailed operating processes were as follows:
200 µL of nanoparticles containing 5 µg Luciferase-mRNA or the drug-loaded nanoparticles prepared above were intraperitoneally injected into 6-8 week-old C57BL/6 mice, and 200 µL of 1× PBS solution containing 3 mg D-Luciferin potassium salt was intraperitoneally injected into the mice 6 hours later. After waiting for 10 minutes, an in vivo mRNA expression level of the m-RNA was evaluated by bioluminescence imaging.

**Table 12 In vivo expression level of mRNA-loaded nanoparticles prepared by the compound of Example 2 after intraperitoneal injection**

| Name of the cationic lipid analogue | Fluorescence intensity of spleen | Structural formula |
|---|---|---|
| A1I4R22C18-2 | 6.48E+06 | 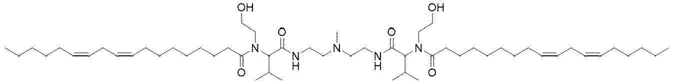 |

### Example 13: An animal experiment for detecting the in vivo expression level of mRNA delivered by tracheal injection

Luciferase-mRNA was used as an mRNA model; and the cationic lipid analogue A1I4R22C18-2 of Example 2 was used to prepare drug-loaded nanoparticles according to the method of Example 6.

Detailed operating processes were as follows:
100 µL of nanoparticles containing 5 µg Luciferase-mRNA or the drug-loaded nanoparticles prepared above were tracheally injected into 6-8 week-old C57BL/6 mice, and 200 µL of 1× PBS solution containing 3 mg D-Luciferin potassium salt was intraperitoneally injected into the mice 6 hours later. After waiting for 10 min, an *in vivo* mRNA expression level was evaluated by bioluminescence imaging.

**Table 13 In vivo expression level of mRNA-loaded nanoparticles prepared by the compound of Example 2 after tracheal injection**

| Name of the cationic lipid analogue | Fluorescence intensity of spleen | Structural formula |
|---|---|---|
| A1I4R22C18-2 | 2.54E+05 | 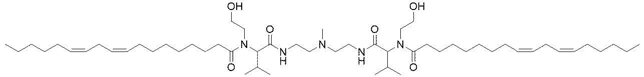 |

### Example 14: An animal experiment for evaluating the immune performance of mRNA delivery in vivo

OVA-mRNA was used as an mRNA model; the cationic lipid analogues of Examples 1 and 2 were used to prepare drug-loaded nanoparticles according to the method of Example 6.

Detailed operating processes were as follows:
C57BL/6 mice aged 6 to 8 weeks were selected for experiments in specific pathogen free (SPF) animal rooms, and 5 mice were selected for each group. 100 µL of nanoparticles containing 5 µg OVA-mRNA or the drug-loaded nanoparticles prepared above were injected into the leg muscle or abdominal cavity of each mouse. After repeating once with the same dose for enhanced immunization, blood was collected for antibody titer analysis by enzyme-linked immunosorbent assay (ELISA).

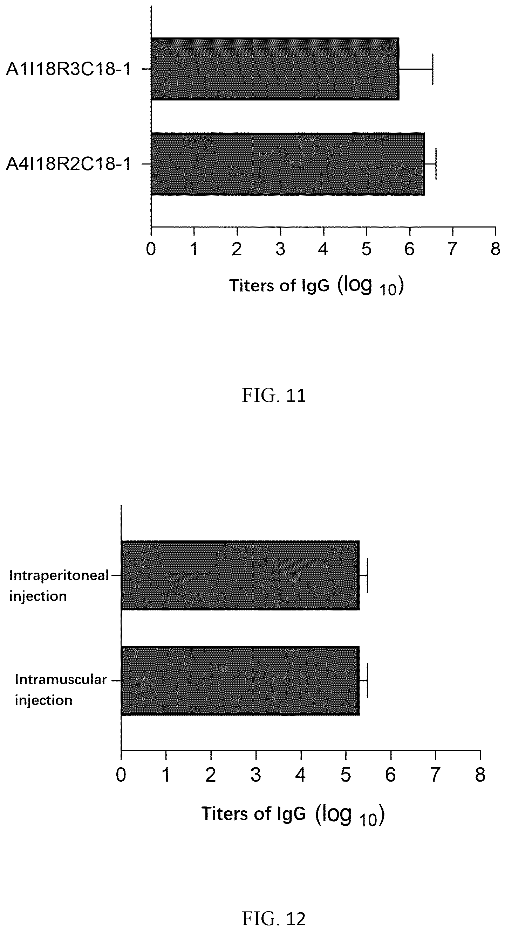

The cationic lipid analogue in Example 1 was taken as an example. After the nanoparticles containing 5 µg OVA-mRNA or the drug-loaded nanoparticles prepared above were intramuscularly injected on Day 0 and Day 14, blood was collected on Day 21 for antibody titer analysis. The results were shown in Figure 11.

The cationic lipid analogue A1I4R22C18-2 in Example 2 was taken as an example. After the nanoparticles containing 5 µg OVA-mRNA or the drug-loaded nanoparticles prepared above were injected on Day 0 and Day 7 by intramuscular injection or by intraperitoneal injection, blood was collected on Day 14 for antibody titer analysis, and the results were shown in Figure 12.

### Example 15: An animal experimenting for detecting the in vivo delivery level of siRNA by intravenous injection

Cy5-siRNA was used as a siRNA model; and the cationic lipid analogues A4I18R2C18-2 and A1I4R22C18-2 in Example 1 were used to prepare drug-loaded nanoparticles according to the method of Example 6.

Detailed operating processes were as follows:
200 µL of nanoparticles containing 10 µg Cy5-siRNA or the drug-loaded nanoparticles prepared above were injected into the leg veins of 6-8 week-old C57BL/6 mice by intravenous injection, and the *in vivo* delivery level of siRNA was evaluated by bioluminescence imaging 1 hour later.

**Table 14 In vivo delivery level of siRNA-loaded nanoparticles prepared by the compound of Example 1 after intravenous injection**

| Name of the cationic lipid analogue | Fluorescence intensity | | | | |
|---|---|---|---|---|---|
| | Heart | Liver | Spleen | Lung | Kidney |
| A4I18R2C18-2 | 9.93E+08 | 5.59E+10 | 4.28E+09 | 9E+09 | 6.11E+10 |

| Structural formula | | | | | |
|---|---|---|---|---|---|
| 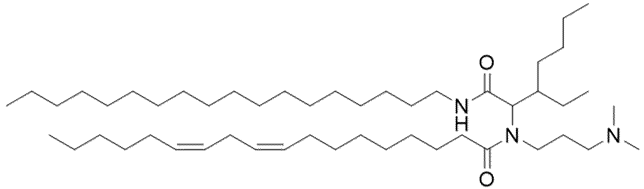 | | | | | |

**Table 15 In vivo delivery level of siRNA-loaded nanoparticles prepared by the compound of Example 2 after intravenous injection**

| Name of the cationic lipid analogue | Fluorescence intensity | | | | |
|---|---|---|---|---|---|
| | Heart | Liver | Spleen | Lung | Kidney |
| A1I4R22C18-2 | 2.24E+09 | 1.73E+11 | 1.49E+10 | 1.2E+10 | 2.25E+ 10 |

| Structural formula | | | | | |
|---|---|---|---|---|---|
| 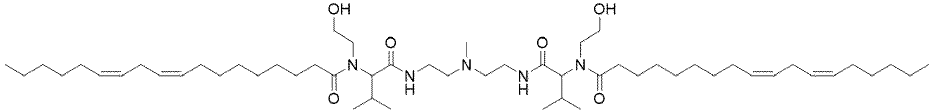 | | | | | |

Finally, it should be noted that the above embodiments are only intended to illustrate the technical solutions of the present disclosure, rather than to limit the protection scope of the present disclosure. Although the present disclosure has been described in detail with reference to the preferred embodiments, those skilled in the art should understand that the technical solutions of the present disclosure can be modified or equivalently replaced, without departing from the essence and scope of the technical solutions of the present disclosure.

## Claims

1. A cationic lipid analogue with a structure of any one of formula (I) to formula (V): 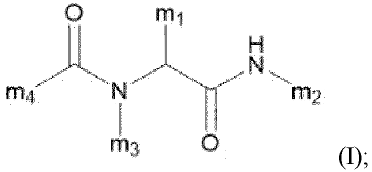 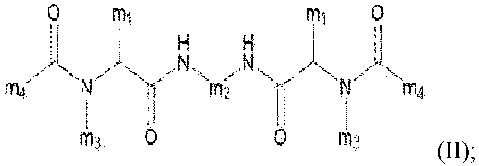 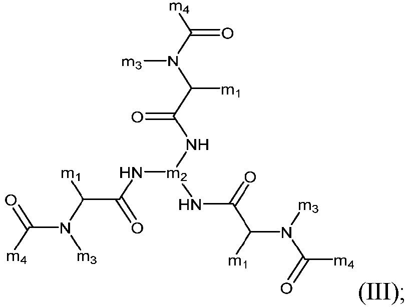 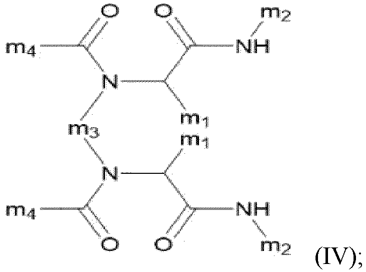 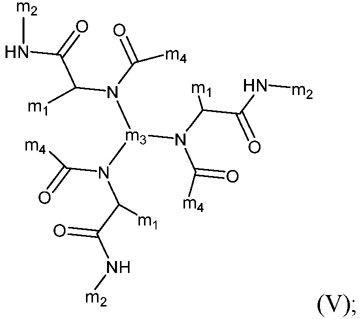
wherein each of m₁ is independently at least one selected from the group consisting of a hydrogen atom, a straight-chain alkyl, a branched-chain alkyl, a straight-chain alkenyl, a branched-chain alkenyl, a substituted alkynyl, a cycloalkyl, a phenyl, and a heteroatom-containing aromatic group;
each of m₂ is independently at least one selected from the group consisting of a straight-chain alkyl, a straight-chain alkenyl, a heteroatom-containing straight-chain alkyl, a heteroatom-containing straight-chain alkylene, a heteroatom-containing branched-chain alkyl, a heteroatom-containing branched-chain alkylene, a heterocycle-containing alkyl, an heterocycle-containing alkylene, an ester alkyl, and an ether alkyl;
each of m₃ is independently at least one selected from the group consisting of a straight-chain alkyl, a straight-chain alkenyl, a cycloalkyl, a heteroatom-containing straight-chain alkyl, a heteroatom-containing branched-chain alkyl, a heteroatom-containing branched-chain alkylene, a heterocycle-containing alkyl, a heteroatom-containing and aromatic ring-containing alkyl, a hydroxyl-containing alkyl, an ester alkyl, an ether alkyl, and a sulfonate substituted alkyl; and
each of m₄ is independently at least one selected from the group consisting of a straight-chain alkyl, a branched-chain alkyl, a straight-chain alkenyl, a branched-chain alkenyl, a straight-chain alkynyl, a heteroatom-containing straight-chain alkyl, a heteroatom-containing branched-chain alkyl, and a heterocycle-containing alkyl.

2. The cationic lipid analogue according to claim 1, wherein in the formula, each of m₁ is independently selected from the group consisting of 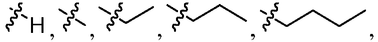 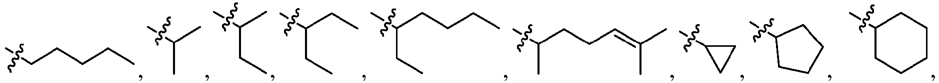 and 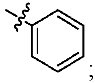 and/or
each of m₂ is independently selected from the group consisting of 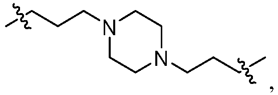 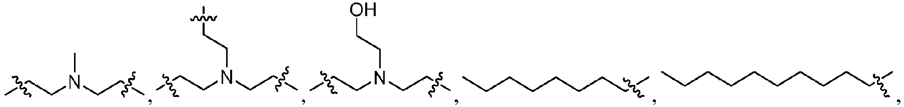 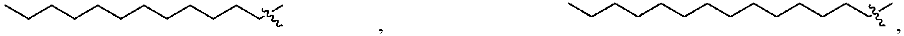 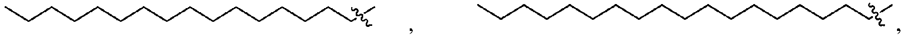 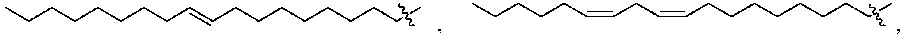 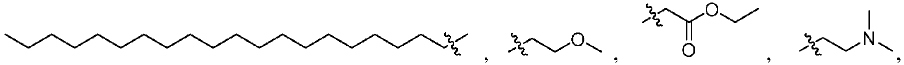 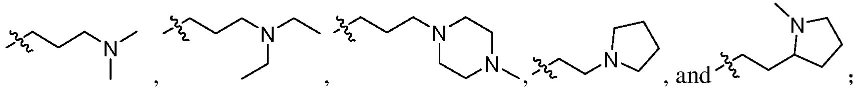 and/or
each of m₃ is independently selected from the group consisting of 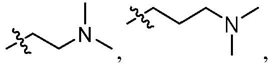 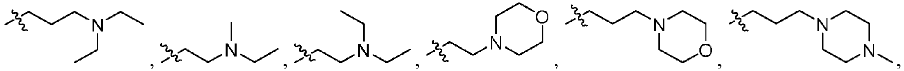 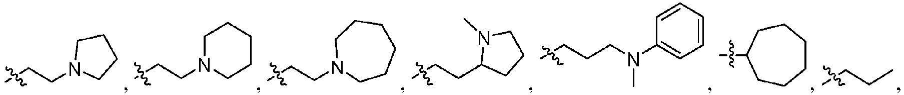 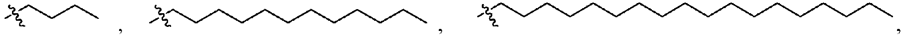 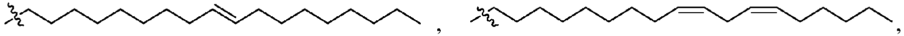 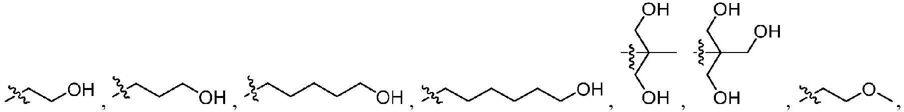 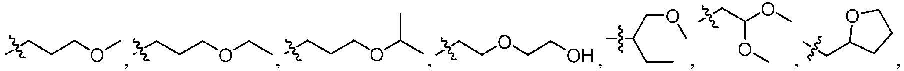 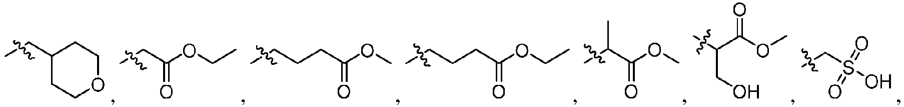 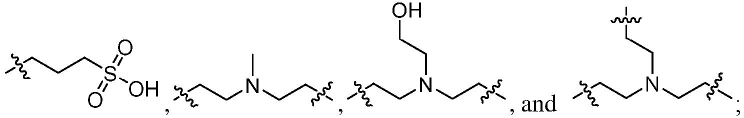 and/or
each of m₄ is independently selected from the group consisting of

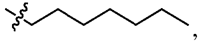

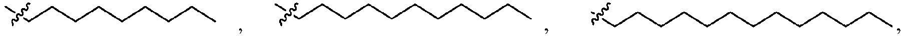

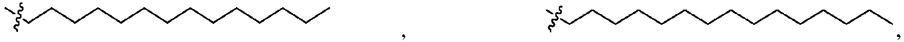

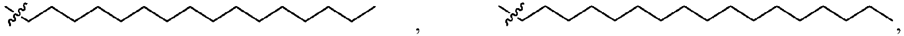

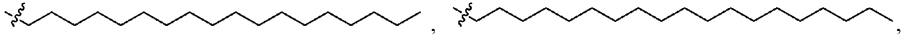

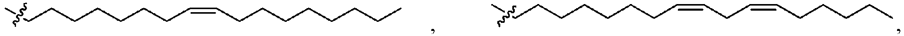

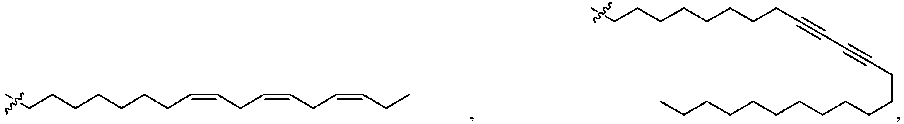

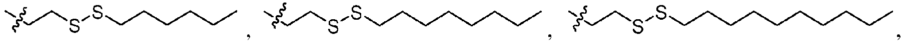

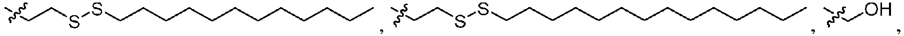

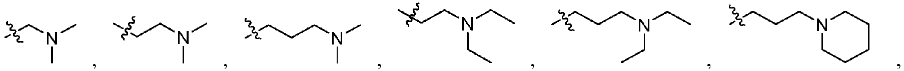

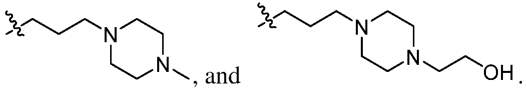

3. The cationic lipid analogue according to claim 2, wherein each of m₁ is independently selected from the group consisting of 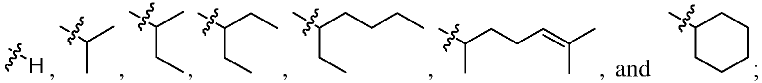 and/or
each of m₂ is independently selected from the group consisting of 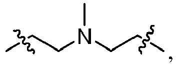 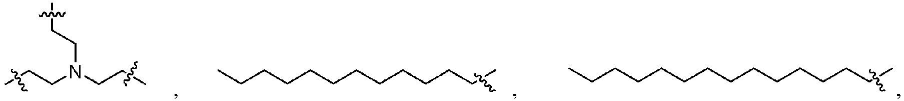 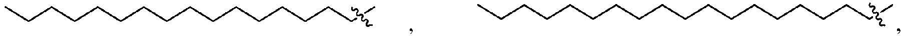 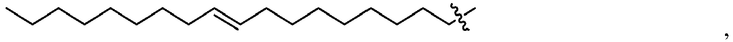 and 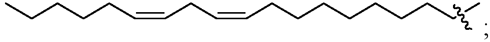 and/or
each of m₃ is independently selected from the group consisting of 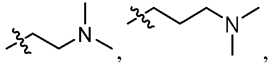 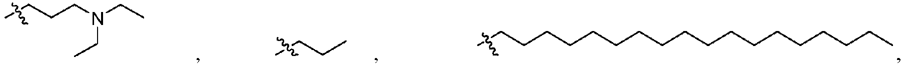 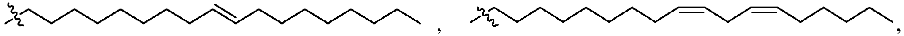 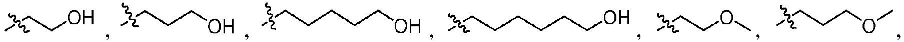 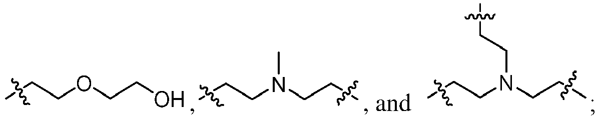 and/or
each of m₄ is independently selected from the group consisting of

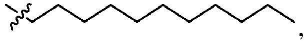

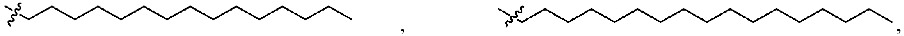

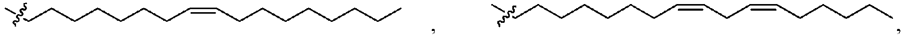

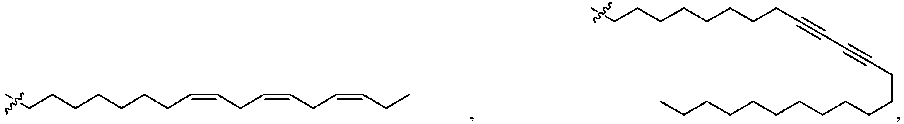

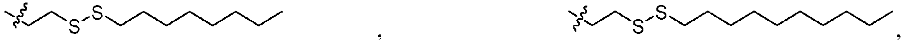

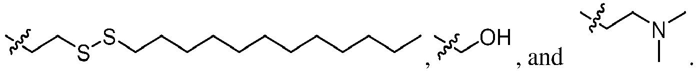

4. A method for preparing the cationic lipid analogue according to any of the claims 1-3, wherein the method comprises: adding an aldehyde and an amine to an organic solution; allowing to react for 10-120 minutes before adding a carboxylic acid and an isonitrile in sequence; allowing to react at 4-60 °C for 6-72 hours; and performing a separation and a purification to obtain a product.

5. The method for preparing the cationic lipid analogue according to claim 4, wherein a molar ratio of the aldehyde, the amine, the carboxylic acid and the isonitrile is (0.1-1.5): (0.1-1.5): (0.1-1.5): (0.1-1.5).

6. The method for preparing the cationic lipid analogue according to claim 4, wherein the isonitrile is one of a monofunctional isonitrile, a bifunctional isonitrile, or a trifunctional isonitrile.

7. The method for preparing the cationic lipid analogue according to claim 5, wherein the isonitrile is any one of the following compounds, I3 to I38:
| | | | | | |
|---|---|---|---|---|---|
| I3 | 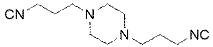 | I8 | 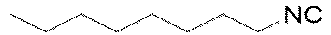 | I31 | 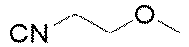 |
| I4 | 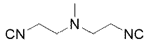 | I10 | 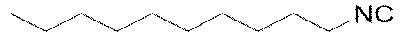 | I32 | 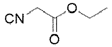 |
| | | I12 | 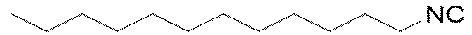 | | |
| I5 | 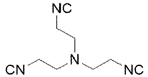 | I14 | 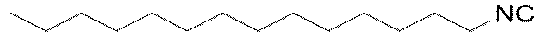 | I33 | 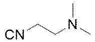 |
| | | I16 | 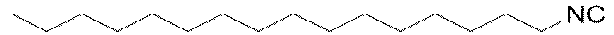 | | |
| I6 | 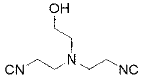 | I18 | 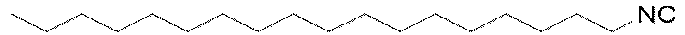 | I34 | 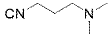 |
| | | I18-1 | 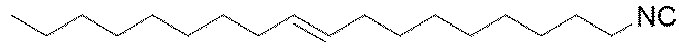 | I35 | 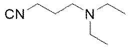 |
| | | I18-2 | 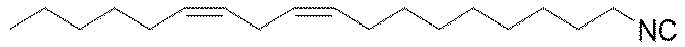 | | |
| | | I20 | 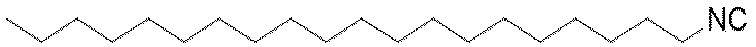 | I36 | 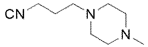 |
| | | | | I37 | 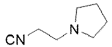 |
| | | | | I38 | 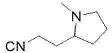 |

8. The method for preparing the cationic lipid analogue according to claim 4, wherein the aldehyde is a monofunctional aldehyde.

9. The method for preparing the cationic lipid analogue according to claim 8, wherein the aldehyde is any one of the following compounds, A1 to A35:
| | | | | | |
|---|---|---|---|---|---|
| A1 | 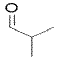 | A21 | 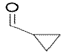 | A30 | 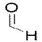 |
| A2 | 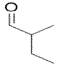 | A22 | 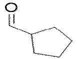 | A31 | 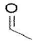 |
| A3 | 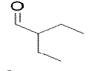 | A23 | 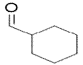 | A32 | 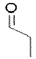 |
| A4 | 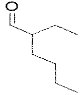 | A24 | 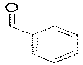 | A33 | 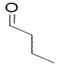 |
| A5 | 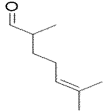 | | | A34 | 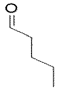 |
| | | | | A35 | 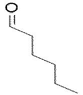 |

10. The method for preparing the cationic lipid analogue according to claim 4, wherein the amine is one of a monofunctional amine, a bifunctional amine, or a trifunctional amine.

11. The method for preparing the cationic lipid analogue according to claim 10, wherein the amine is any one of the following compounds, R1 to R53:
| | | | | | | | |
|---|---|---|---|---|---|---|---|
| R1 | 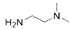 | R21 | 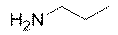 | R37 | 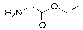 | R46 | 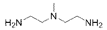 |
| R2 | 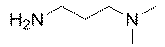 | R22 | 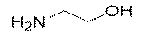 | R38 | 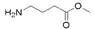 | R47 | 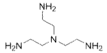 |
| R3 | 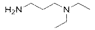 | R23 | 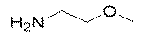 | R39 | 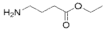 | | |
| R4 | 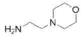 | R24 | 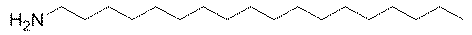 | | | | |
| | | R25 | 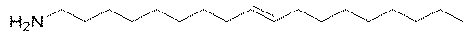 | R40 | 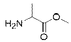 | R48 | 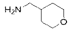 |
| R5 | 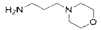 | R26 | 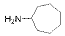 | | | | |
| R6 | 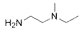 | R27 | 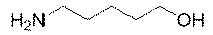 | R41 | 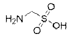 | R49 | 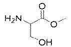 |
| R7 | 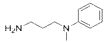 | R28 | 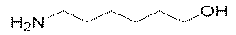 | R42 | 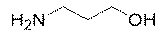 | R50 | 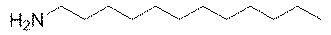 |
| R8 | 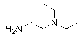 | R29 | 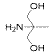 | R43 | 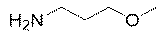 | R51 | 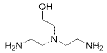 |
| R9 | 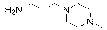 | | 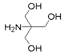 | R44 | 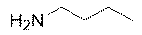 | | |
| R10 | 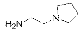 | R30 | | R45 | 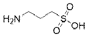 | R53 | 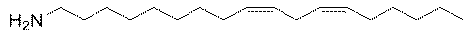 |
| R11 | 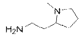 | R31 | 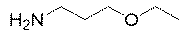 | | | | |
| | | | 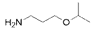 | | | | |
| R12 | 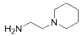 | R32 | 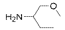 | | | | |
| R13 | 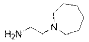 | R33 R34 | 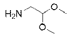 | | | | |
| | | R35 | 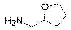 | | | | |
| | | R36 | 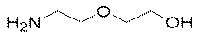 | | | | |

12. The method for preparing the cationic lipid analogue according to claim 4, wherein the carboxylic acid is any one of the following compounds, C8 to C30, or S11 to S19:
| | | | | | |
|---|---|---|---|---|---|
| C8 | 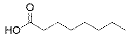 | C21 | 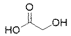 | S11 | 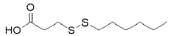 |
| C10 | 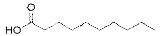 | | | | |
| C12 | 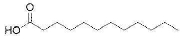 | C22 | 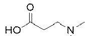 | S13 | 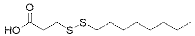 |
| C14 | 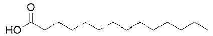 | | | S15 | 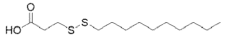 |
| C15 | 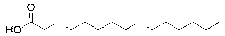 | C24 | 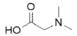 | | |
| C16 | 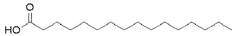 | C25 | 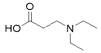 | S17 | 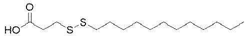 |
| C17 | 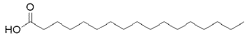 | | | S19 | 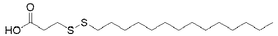 |
| C18 | 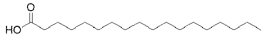 | C26 | 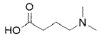 | | |
| C19 | 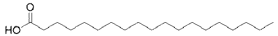 | C27 | 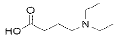 | | |
| C20 | 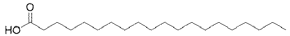 | | | | |
| C18-1 | 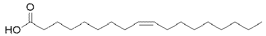 | C28 | 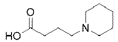 | | |
| C18-2 | 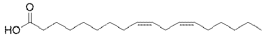 | C29 | 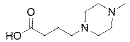 | | |
| C18-3 | 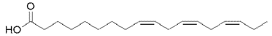 | | | | |
| C23 | 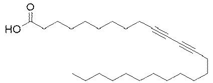 | C30 | 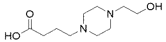 | | |

13. A composition comprising the cationic lipid analogue according to any one of claims 1-3, wherein the composition comprises the cationic lipid analogue and at least one selected from the group consisting of a sterol, an auxiliary lipid, and a polyethylene glycol lipid derivative.

14. The composition comprising the cationic lipid analogue according to claim 13, wherein the sterol comprises at least one selected from the group consisting of a cholesterol, a sitosterol, a stigmasterol, and a cholesterol derivative; the auxiliary lipid is a neutral phospholipid.

15. The composition comprising the cationic lipid analogue according to claim 13, wherein a molar ratio of the cationic lipid analogue, the cholesterol, the auxiliary lipid, and the polyethylene glycol lipid derivative is (20-70): (20-50): (2-30): (0.1-20).

16. A drug-loaded nanoparticle, comprising the composition comprising the cationic lipid analogue according to claim 13 and a drug.

17. The drug-loaded nanoparticle according to claim 16, wherein the drug comprises at least one selected from the group consisting of a small molecule compound, a nucleic acid molecule, a protein or polypeptide molecule, and a gene editing complex.

18. The drug-loaded nanoparticle according to claim 17, wherein the nucleic acid molecule is at least one selected from the group consisting of messenger RNA, transfer RNA, dsRNA, shRNA, DNA, plasmid DNA, siRNA, antisense oligonucleotide, aiRNA, and miRNA; the gene editing complex is mRNA/sgRNA or Cas9/sgRNA.

19. The drug-loaded nanoparticle according to claim 17, wherein a mass ratio of the cationic lipid analogue to the nucleic acid molecule is (2~50): 1.

20. A method for preparing the drug-loaded nanoparticle according to claim 19, comprising following steps:
(1) dissolving the composition comprising the cationic lipid analogue in an organic solution to obtain an organic phase;
(2) dissolving the nucleic acid molecule in a buffer solution to obtain an aqueous phase; a volume ratio of the aqueous phase to the organic phase is (2-5): 1;
(3) rapidly mixing the aqueous phase and the organic phase evenly to obtain a mixture, and dialyzing the mixture to obtain the drug-loaded nanoparticle.

21. A use of the cationic lipid analogue according to any one of claims 1-3, the composition comprising the cationic lipid analogue according to any one of claims 13-15, or the drug-loaded nanoparticle according to any one of claims 16-19 in preparing, delivering or transporting a drug molecule or a nucleic acid vaccine.
